# EUROPÄISCHE PATENTSCHRIFT

(11) **EP 1 480 659 B1**
(45) Veröffentlichungstag und Bekanntmachung des Hinweises auf die Patenterteilung: **10.10.2007**
(21) Anmeldenummer: 03706119.9
(22) Anmeldetag: 26.02.2003
(51) Int. Cl.: A61K 33/06, A61P 7/00, A23K 1/175, A23L 1/304

(54) **VERWENDUNG VON ZEOLITHEN ZUR SENKUNG DER ANTEILE VON LACTATEN IM MENSCHLICHEN UND TIERISCHEN ORGANISMUS**
USE OF ZEOLITHES FOR REDUCING THE PROPORTION OF LACTATES IN HUMAN AND ANIMAL ORGANISMS
UTILISATION DE ZEOLITES POUR REDUIRE LES PROPORTIONS DE LACTATES DANS LES ORGANISMES HUMAINS ET ANIMAUX

(30) Priorität: 26.02.2002 AT 2942002
(43) Veröffentlichungstag der Anmeldung: 01.12.2004
(73) Patentinhaber: Hraschan, Jakob, 9580 Drollobach (AT)
(72) Erfinder: HERZOG, Christian, 8010 Graz (AT); HRASCHAN, Jakob, 9580 Drollobach (AT); LELAS, Antonio, A-9585 Villach-Gödersdorf (AT)
(74) Vertreter: Patentanwälte BARGER, PISO & PARTNER
(86) Internationale Anmeldenummer: PCT/AT2003/000059
(87) Internationale Veröffentlichungsnummer: WO 2003/072116

(56) Entgegenhaltungen:
- G.C.SHURSON E.A.: "Effects of zeolite or clinoptilolite in diets of growing swine" JOURNAL OF ANIMAL SCIENCE, Bd. 59, Nr. 6, 1984, Seiten 1536-1545, XP008018449
- L.ROMAN E.A.: "Decreased absorption of orally administered ammonia by clinoptilolite" PROCEEDINGS OF THE SOCIETY FOR EXPERIMENTAL BIOLOGY AND MEDICINE, Bd. 166, Nr. 3, 1981, Seiten 369-373, XP008018569
- W.G.POND: "Protection against acute ammonia toxicity by clinoptilolite in mature sheep " NUTRITION REPORTS INTERNATIONAL, Bd. 30, Nr. 4, 1984, Seiten 991-1002, XP008018450
- DATABASE WPI , 1991 Derwent Publications Ltd., London, GB; AN 1991-062527 XP002245058 KURARAY: "Ammonia removal composition for blood plasma-consists of solid esp. zeolite coated polymer , having specified surface pH and fine pore diameter" & JP 03 012172 A (KURARAY), 21. Januar 1991 (1991-01-21)
- PATENT ABSTRACTS OF JAPAN vol. 0161, no. 64, 1992 & JP 04 013633 A (TOMIO NAKAMURA), 17. Januar 1992 (1992-01-17)

## Beschreibung

Die Erfindung betrifft die Senkung der Anteile von Lactaten im menschlichen bzw. tierischen Organismus durch die orale Verabreichung von Zeolithen.

Die Adsorptionsfähigkeit von Zeolithen ist beispielsweise aus der DE 44 03 987 A bekannt. Diese Druckschrift offenbart die Abtrennung von Hydroxymonocarbonsäuren bzw. Hydroxytricarbonsäuren aus wässerigen Lösungen durch derartige Adsorption.

In der US 5,149,435 wird Zeolith als Mittel zur Aufnahme unerwünschter Partikel in wässrigen Medien, darunter auch Blut und Blut-Plasma, angesehen, wodurch ein Filtereffekt erzielt werden soll. In der Publikation von Boranic M.: "What a physician should know about zeolites", Lijec Vjesn. 2000 Nov-Dec; 122(11-12):292-8., wird davon ausgegangen, dass Zeolith bei physiologischen pH-Werten eine nur geringe Löslichkeit aufweist. Davon ausgehend wird in dieser Druckschrift die Ansicht vertreten, dass nur geringe Mengen an freiem Aluminium oder Silizium vom Darm resorbiert werden.

Aus der US 4,537,771 A ist die Verwendung von Zeolithen als Antiacidum zur Behandlung von überschüssiger Magensäure und damit einher gehender Beschwerden bekannt.

Aus dem Abstract der JP 4013633 A ist es bekannt, zur Vermeidung von Verdauungsproblemen und zur Verbesseung der Schönheit der Haut, der Nahrung Zeolithe zuzusetzen.

Aus dem Abstract der JP 56155644 A ist es bekannt, zur Schaffung eines Adsorbents mit verbesserter Verträglichkeit, das Adsorbens mit einer Schichte aus einem speziellen, wasserlöslichem Polymer zu überziehen. Über die Natur des Adsorbens wird in dieser Druckschrift aber nichts ausgeführt.

Aus dem Abstract der JP 62145022 A ist die Verwendung von Zeolithen als Medikament zur Behandlung von Lebensmittelvergifiungen bekannt.

Aus dem Abstract der JP 11116507 A ist die Verwendung modifizierter Zeolithe zur Herstellung von Medikamenten, welcher Art wird aber nicht genannt, bekannt.

Aus dem Abstract der JP 03012172 A ist bekannt, dass zur Behandlung hyperammonischer Personen Zeolithe mit einer Körnung zwischen 0,2 und 5 mm verwendet werden können. Eine kleinere Körnung wird als nachteilig angesehen.

Aus der GB 1 057 044 A ist bekannt, dass Zeolithe verwendet werden können, um Härtemittel für das Aushärten organischer Harze in die Reaktionszone zu transportieren und dort freizusetzen.

G.C. Shurson E.A. hat in "Effects of zeolite or clinoptilolite in diets of growing swine", Journal of animal Science, Bd. 59, Nr. 6, 1984, Seiten 1536-1545 darauf hingewiesen, dass die Verabreichung von Zeolithen das Wachstum der Schweine positiv beeinflußt und dass es im Verdauungstrakt möglicherweise zur Bindung von Ammoniak an den Zeolithen kommt.

L. Roman E.A hat in "Decreased absorption of orally administered ammonia by clinoptilolite in rats", Proceedings of the society for experimental biology and medicine, Bd. 166, Nr. 3, 1981, Seiten 369-373 festgestellt, dass bei Ratten orale Gaben von Zeolith die Aufnahme von oral verabreichtem (NH₄)₂CO₃ reduzieren.

W.G. Pond hat in "Protection against acute ammonia toxicity by clinoptilolite in mature sheep", Nutrition reports international, Bd. 30, Nr. 4, 1984, Seiten 991-1002, die gleiche Erkenntnis bei der oralen Verabreichung von Harnstoff an Schafen gemacht.

Von verschiedenen sogenannten Verzehrprodukten (Nahrungsergänzungen), nämlich gemahlenen Zeolithen, insbesondere Klinoptilolith, ist deren allgemeine Ad- bzw. Absorptionseingenschaft und die Fähigkeit, gepufferte Systeme zu neutralisieren, bekannt.

Es wurde von den Erfindern bei Verabreichung an befreundete Hobby- und Profisportler festgestellt, dass sich die Leistungsfähigkeit, Ausdauer und Regeneration nach Anstrengung durch die Einnahme von Zeolithen in Form von Pulver und Kapseln deutlich verbessert hat.

Es wurde in der Folge mit Sportwissenschaftern Kontakt aufgenommen und dabei konnte in ersten Anwendungsbeobachtungen festgestellt werden, dass sich die Messwerte bei der Ergometrie betreffend des Lactatanteiles überraschend stark verbessert haben.

Es ist allgemein bekannt, dass ein hoher Lactat- bzw. Ammoniumanteil im Organismus (Blut) sich negativ auf die Leistungsfähigkeit von Mensch und Tier auswirkt.

Lactate sind die Salze der Milchsäure. Milchsäure: 2-Hydroxypropionsäure, aliphatische Hydroxycarbonsäure, CH3-CH(OH)-COOH; hat ein asymmetrisches Kohlenstoffatom und tritt deshalb in zwei optisch aktiven Formen (D- und L-Milchsäure) auf. Im menschlichen Körper wird die rechtsdrehende L(+)-Milchsäure sehr schnell umgesetzt, während die linksdrehende D(-)-Milchsäure nur langsam umgebaut wird. Lediglich im Stoffwechsel des Säuglings kann D(-)-Milchsäure noch nicht abgebaut werden und sich deshalb anreichern. Man kennt die Gärungsmilchsäure, die bei der Milchsäuregärung von saurer Milch (aus dem Milchzucker), aus Stärke und Traubenzucker sowie bei der Säuerung pflanzlichen sind, besetzt. Die Kanäle sind groß genug um Gastmoleküle passieren zu lassen. Wasserreiche Phasen können dehydrieren; die Entwässerung erfolgt meist bei Temperaturen unter etwa 400 °C und ist größtenteils umkehrbar. Das Kristallgitter kann durch (OH,F)-Gruppen unterbrochen sein; diese besetzen eine Tetraederspitze, die nicht mit einem benachbarten Tetraeder verbunden ist.

### Struktur und Eigenschaften des Klinoptilolithes:

Die Struktur des Klinoptiloliths beruht auf dem dreidimensionelen Gitter von Tetraedern (SiO₄)⁴⁻, die durch Sauerstoffatome miteinander gebunden werden, wobei ein Teil von Siliziumatomen durch Aluminiumatome (AlO₄)⁵⁻ ersetzt wird. Partielle Ersetzung von Ionen Si⁴⁺ durch Ionen Al³⁺ erregt Ladungsdefizit und dieses wird durch die Kationen (der Alkali- und Erdalkalimetalle) kompensiert, die bestimmte Stellungen im Netz von Kanälen und Poren besetzen. Ausmasse von Kanälen sind gross genug, damit in sie Moleküle von Grösse ein paar Zehntel von Nanometer hineindringen können und adsorbiert werden. Das Totalvolumen dieser Hohlkanäle beträgt 24-32 % des Gesamtvolumens.

Die Eigenschaften von Zeolithen folgen aus den Besonderheiten ihrer kristallischer Struktur. Diese Besonderheit liegt darin, dass der räumliche Molekülaufbau Kanäle und Höhlen mit konstanter Grösse bildet. In diesen Kanälen können die Stoffe vom festen, tropfbarflüssigen und gasförmigen Aggregatzustand aufgefangen werden. Wie die überwiegende Zahl von Silikaten ist auch der vorliegende Naturstoff inert, d.h. er reagiert weder mit Nahrungskomponenten oder deren Metaboliten noch mit Sekreten oder symbiotischen Stoffwechselprodukten im chemischen Sinne. Seine Wirkungen beruhen auf physikalischen Kräften (Oberflächen-Adhäsion, elektromagnetisches Kraftfeld der Einzelkristalle, Van der Waal'sche Kraft u.a.)

### Abbau und Verarbeitung des Rohstoffes:

Der geologische Aufbau der bevorzugt genutzen Lagerstätte in der Sedimentationsschichte des Flachlandes in der Ostslowakei ist einfach. Sie besteht aus einer Lage des Teolith-Tuffs mit einer Stärke von 90-115 m. Auf der Oberfläche tritt sie in einer Länge von ca. 520 m aus. Die hydrogeologischen Verhältnisse der Lagerstätte sind ebenfalls einfach. Der überwiegende Teil der Lagerstätte ist bewässert. Der Grossteil der Vorräte kann im Wand-Die beschriebenen Wirkungen werden durch orale Verabreichung (Kapseln zum Schlucken oder Pulver trocken eingenommen bzw. in Flüssigkeit gelöst und getrunken) erzielt. Für die orale Verabreichung eignen sich neben Tabletten, Kapseln oder Pulver auch Nahrungs- bzw. Futtermittel aus oder auf Basis von Zeolithen herzustellen.

Mögliche Einsatzgebiete neben der pharmazeutischen und verzehrproduktgemäßen Anwendung sind die Lebensmittelindustrie und die Landwirtschaft sowie der Bereich Sport (körperliche Ertüchtigung bzw. Leistungssteierung) für Mensch und Tier.

### Informationen zum Mineralstoff Zeolith:

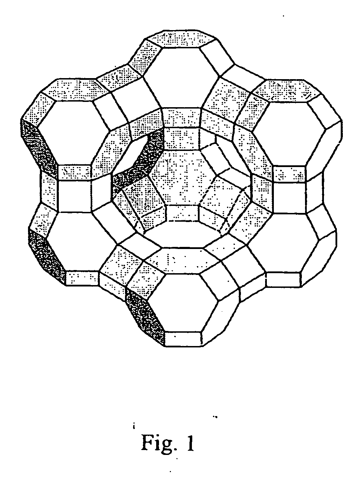

Ein Zeolith ist eine kristalline Substanz, deren Struktur durch ein Kristallgitter aus miteinander verbundenen Tetraedern, jeder bestehend aus einem Kation und vier SauerstoffAtomen, charakterisiert wird. Eine solche Struktur ist in Fig. 1 beispielhaft dargestellt. Dieses Kristallgitter enthält offene Hohlräume in Form von Käfigen und Kanälen. Diese sind gewöhnlich durch H2O- Moleküle und zusätzliche Kationen, welche austauschbar sind, besetzt. Die Kanäle sind groß genug um Gastmoleküle passieren zu lassen. Wasserreiche Phasen können dehydrieren; die Entwässerung erfolgt meist bei Temperaturen unter etwa 400 °C und ist größtenteils umkehrbar. Das Kristallgitter kann durch (OH,F)-Gruppen unterbrochen sein; diese besetzen eine Tetraederspitze, die nicht mit einem benachbarten Tetraeder verbunden ist.

### Struktur und Eigenschaften des Klinoptilolithes:

Die Struktur des Klinoptiloliths beruht auf dem dreidimensionelen Gitter von Tetraedern (SiO₄)⁴⁻, die durch Sauerstoffatome miteinander gebunden werden, wobei ein Teil von Siliziumatomen durch Aluminiumatome (AlO₄)⁵⁻ ersetzt wird. Partielle Ersetzung von Ionen Si⁴⁺ durch Ionen Al³⁺ erregt Ladungsdefizit und dieses wird durch die Kationen (der Alkali- und Erdalkalimetalle) kompensiert, die bestimmte Stellungen im Netz von Kanälen und Poren besetzen. Ausmasse von Kanälen sind gross genug, damit in sie Moleküle von Grösse ein paar Zehntel von Nanometer hineindringen können und adsorbiert werden. Das Totalvolumen dieser Hohlkanäle beträgt 24-32 % des Gesamtvolumens.

Die Eigenschaften von Zeolithen folgen aus den Besonderheiten ihrer kristallischer Struktur. Diese Besonderheit liegt darin, dass der räumliche Molekülaufbau Kanäle und Höhlen mit konstanter Grösse bildet. In diesen Kanälen können die Stoffe vom festen, tropfbarflüssigen und gasförmigen Aggregatzustand aufgefangen werden. Wie die überwiegende Zahl von Silikaten ist auch der vorliegende Naturstoff inert, d.h. er reagiert weder mit Nahrungskomponenten oder deren Metaboliten noch mit Sekreten oder symbiotischen Stoffwechselprodukten im chemischen Sinne. Seine Wirkungen beruhen auf physikalischen Kräften (Oberflächen-Adhäsion, elektromagnetisches Kraftfeld der Einzelkristalle, Van der Waal'sche Kraft u.a.)

### Abbau und Verarbeitung des Rohstoffes:

Der geologische Aufbau der bevorzugt genutzen Lagerstätte in der Sedimentationsschichte des Flachlandes in der Ostslowakei ist einfach. Sie besteht aus einer Lage des Teolith-Tuffs mit einer Stärke von 90-115 m. Auf der Oberfläche tritt sie in einer Länge von ca. 520 m aus. Die hydrogeologischen Verhältnisse der Lagerstätte sind ebenfalls einfach. Der überwiegende Teil der Lagerstätte ist bewässert. Der Grossteil der Vorräte kann im Wandbruch-Verfahren abgebaut werden, die Abwässerung kann dabei mittels Schwerkraftleitung erfolgen, ein kleiner Teil der Vorräte ist nur mittels Schachtbau-Verfahrens abbaubar.

Der Träger der Gebrauchseigenschaften des Rohstoffes ist das Zeolith-Mineral Klinoptilolith, der Durchschnittswert der Lagerstätte beträgt 59,8 Gew.%. Der grundlegende Qualitätsparameter nach den Bedingungen der Nutzbarkeit ist die Teil-Ionenaustauschkapazität, festgestellt mit 0,15 Molar-Lösung NH₄Cl nach STN 72 10 76 (Feststellung der Austauschkapazität und Austausch-Kationen toniger Böden).

Die Qualität des Zeoliths aus dieser Lagerstätte wird nach dem prozentuellen Anteil des Klinoptiloliths bestimmt:

| % vom Klinoptilolith | Anteil des Rohstoffes |
|---|---|
| 70 - 80 | 16,4 % |
| 55 - 70 | 73,0 % |
| 35 - 55 | 10,6 % |

Der Zeolith-Abbau wird qualitativ anhand der Analysenergebnisse gesteuert, die im Rahmen der geologischen Erforschung der Lagerstätte und regelmässiger Bernusterung der Bruchstelle vor dem Abbau durchgeführt werden. Die Analysenergebnisse werden aufgezeichnet und in Grubenkarten gezeichnet.

Der Abbau erfolgt in Sinne der Bedingungen, die durch die Entscheidung über die Genehmigung der bergbaulichen Tätigkeit und der Sprengarbeiten vom kleinen Umfang, die Bergbauzulassung, den Plan des Aufschlusses, der Vorbereitung und Förderung (POPD) festgelegt werden.

Der Zeolith-Abbau im Bruch erfolgt im bergbaulichen Verfahren unter Anwendung von Sprengarbeiten vom kleineren Umfang. Das gelöste Material wird mittels Baggers an LKW aufgeladen und an eine Brechanlage direkt in dem Bruch, transportiert. Mit einem Wagenbeschicker wird das Material an die Lehmabschneider-Grobsortieranlage zugeführt, die aus 2 Sieben mit ø 120 mm und ø 25 mm besteht.

Der Siebrückstand über 120 mm wird mit Schwerkraftleitung an einen Maulbrecher mit einem Ausstrittschlitz von 100 mm zugeführt. Der auf diese Weise aufbereitete Rohstoff wird mittels Bandförderer in zwei stählerne Behälter mit Inhalt von 2 x 300 m³ transportiert. Die Fraktion 25-120 mm fällt direkt an einen kurzen Bandförderer zu dem zerkleinerten Material. Die absortierte Fraktion 0-25 mm (Ton, kleine Zeolith-Partikeln) wird mit einem Förderbandsystem ausserhalb des Bereich der Brechanlage an eine freiliegende Berghalde abtransportiert.

Der zerkleinerte Zeolith wird aus den Behältern vom Bruch mit LKW an einen überdachten Lagerplatz transportiert.

### Trocknung des Zeoliths:

Der Zeolith aus dem überdachten Lagerplatz wird an die Brechlinie transportiert, wo er mit einem Hammerbrecher auf eine Fraktion bis zu 50 mm zerkleinert wird. Aus dem Hammerbrecher wird Zeolith in die Mahlgutbehälter für die Trocknungslinie 2 und 3 gefördert. Der zerkleinerte Zeolith wird aus dem Behälter mit einem Schuppen- und Gurnmibandförderer in den Trockenzylinder gefördert.

In dem rotierenden Trockenzylinder wird das Material mit dem Trocknungsmedium gleichströmig weiter gefördert. Die Einbauten in dem Trockenzylinder sorgen dafür, das das Material gut verschüttet und gleichmässig in dem gesamten Zylinder verteilt wird, wodurch die gleichmässige Trocknung unterstützt wird. Das Trocknungsmedium wird in einer Verbrennungskammer durch die Verbrennung der Staubkohle an einem beweglichen Rost zubereitet.

Für den Zug des Trocknungsmediums sorgt ein hinter dem Elektro-Abscheider aufgestellter Ventilator. Die Verbrennungsluft wird mit dem Ventilator unter den beweglichen Rost eingetrieben. Die Temperatur des Trocknungsmediums am Einritt in den Trockenzylinder liegt bei 400-500°C und am Austritt bei 95-105°C. Die Trocknungstemperatur ist sehr wichtig. Höhere Temperaturen würden zu der fortlaufenden Zerstörung der Kristallstruktur vom Klinoptilolith führen und zu einem teilweisen Verlust der Gebrauchseigenschaften. Der Zeolith wird standardmässig auf eine Austrittsfeuchtigkeit von 4 % getrocknet (bei Bedarf auch weniger). Das Material nach dem Passieren des Trockenzylinders fällt durch eine Rutsche in eine Sammelgrube hinein. Kleinere Materialpartikeln werden in einen Zyklon gezogen, der als ein Grobabschneider dient. Feinste Fraktionen, die im Zyklon nicht erfasst werden, werden von einem Elektrofilter erfasst, wo sie infolge hoher E-lektrospannnung an den Elektroden haften bleiben. Das von dem Zyklon und Elektroabschneider abgefangene Staubmaterial wird in einem Behälter zugeführt.

### Mahlung des Zeoliths:

Für die Mahlung des Zeoliths ist eine Mühle vorgesehen. Beim Bedarf besteht die Möglichkeit, auch weitere Mühlen gleicher Bauweise und Leistungsparameter für die Mahlung des Zeoliths zu verwenden.

Der getrocknete Zeolith wird aus der Sammelgrube unter den Trocknungslinien mittels eines Brückenkrans in einen gekennzeichneten Behälter, situiert über die entsprechende Mühle, gefördert, aus welchem der Zeolith mit einem Harding-Beschicker entnommen und in die Mühle geführt wird. Die Dosiermenge wird so geregelt, dass die Leistungs - und

Güteparameter des Mahlens eingehalten werden. Die Mühlen sind 2-Kammer-Mühlen. Die Füllung der ersten Kammer bilden die Mahlkugeln von Durchmesser 30-100 mm, die Füllung der zweiten Kammer bilden die Mahlkörper - Cylpebse.

Die Kammern sind gegenseitig durch eine Trennwand abgetrennt, die den Durchgang der ungenügend gelösten Materialstücken in die zweite (Nachmahl-) Kammer verhindert. Das Material wird bei dem Durchgang der Mühle mitels Stössen der Mahlkörper auf die gewünschte Feinheit zerkleinert. Das Mahlgut fällt aus der Mühle durch ein Rüttelsieb und flachen Verschluss in den Behälter, aufgestellt über der Füller-Pumpe. Ein Ventilator sorgt für die Belüftung der Mühle, wobei die gelöste Feuchtigkeit des Eingangsmaterials und die durch die Reibung der Mahlkörper freigesetzte Wärme abgeführt wird. Mit der Fuller-Pumpe wird das Mahlgut durch eine Rohrleitung in die Beton-Silos gefördert.

### Versuche:

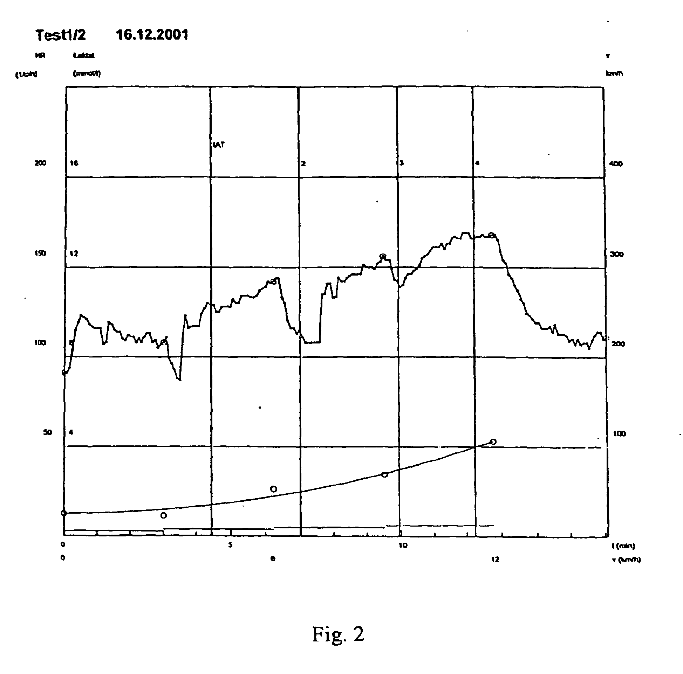

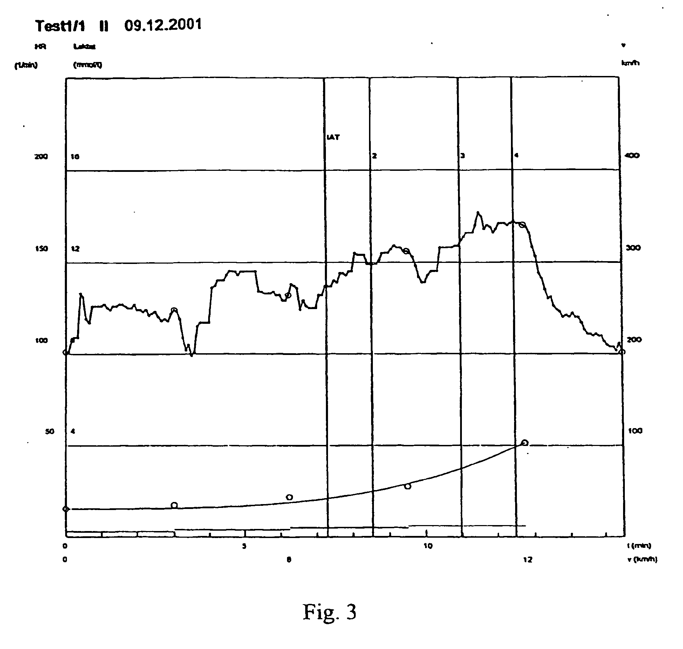

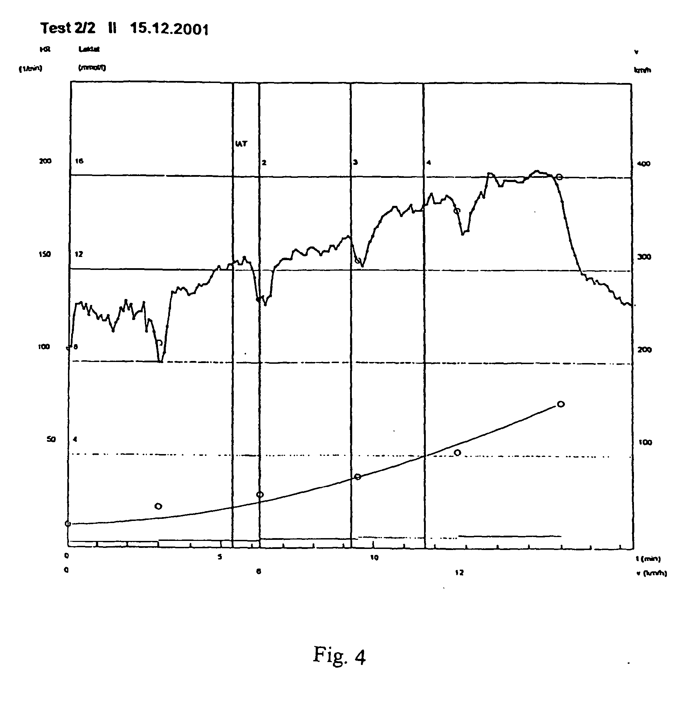

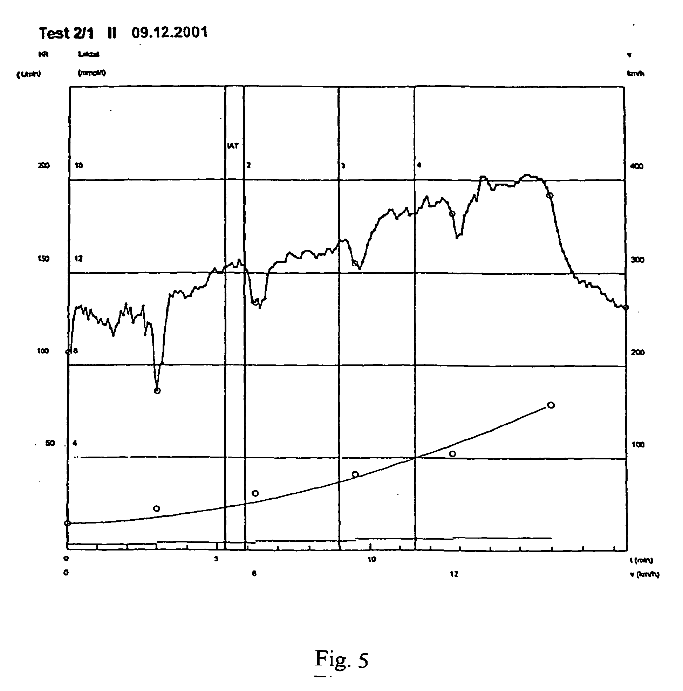

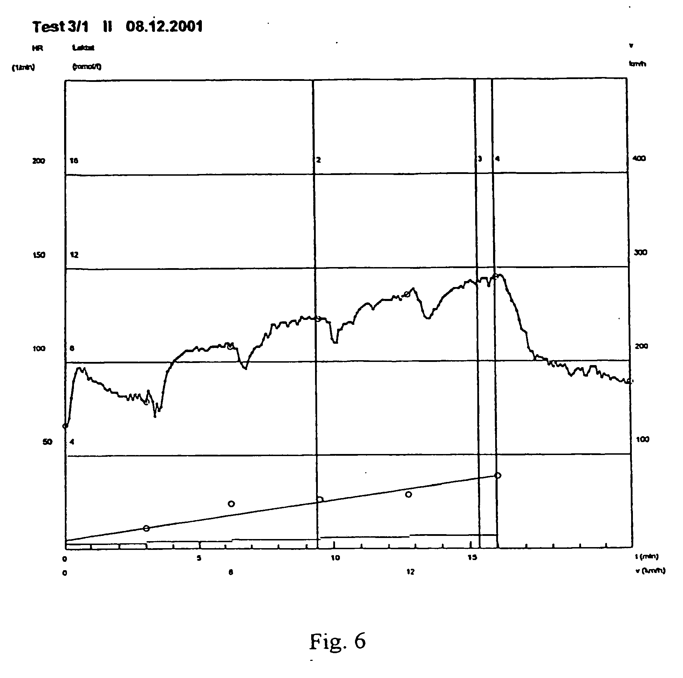

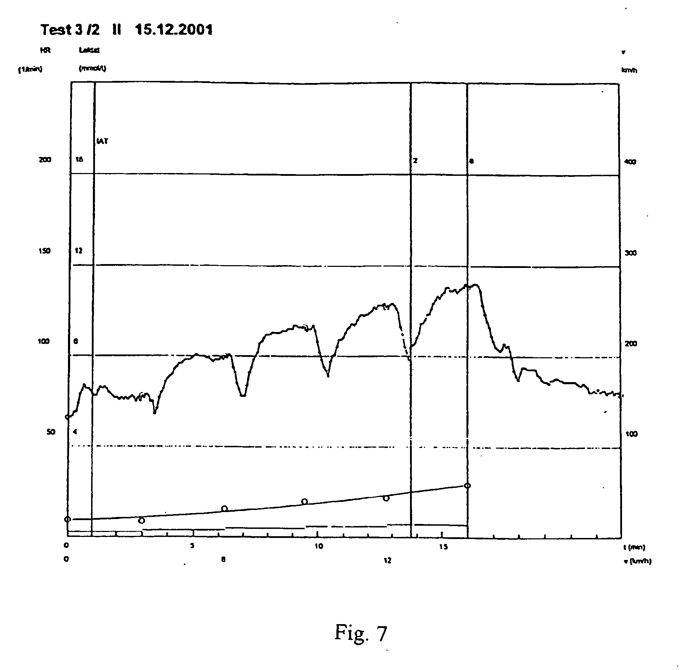

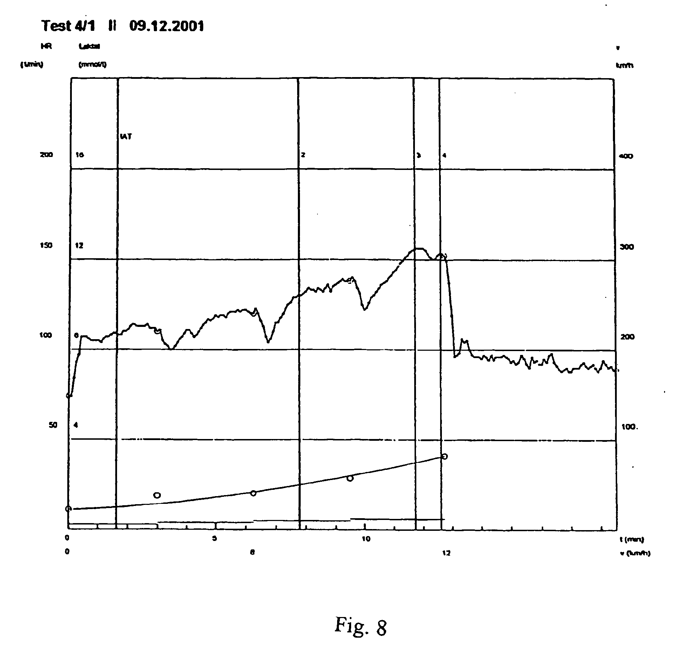

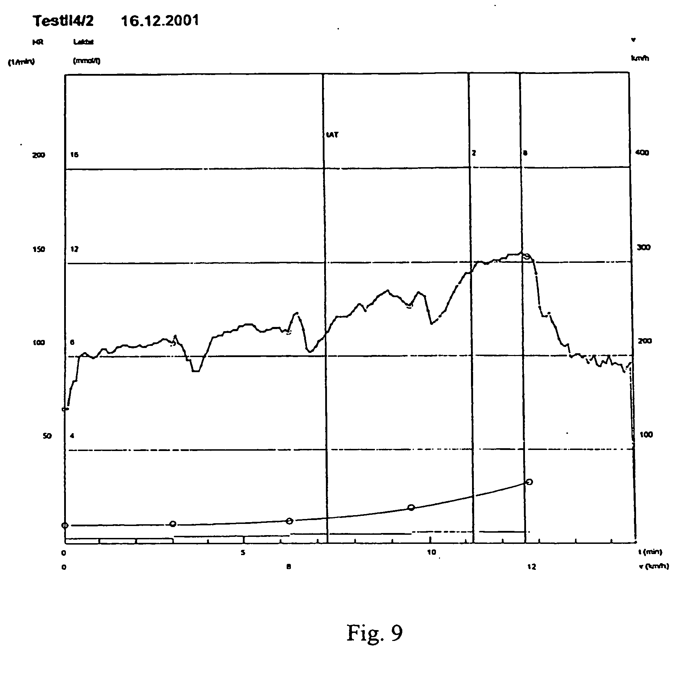

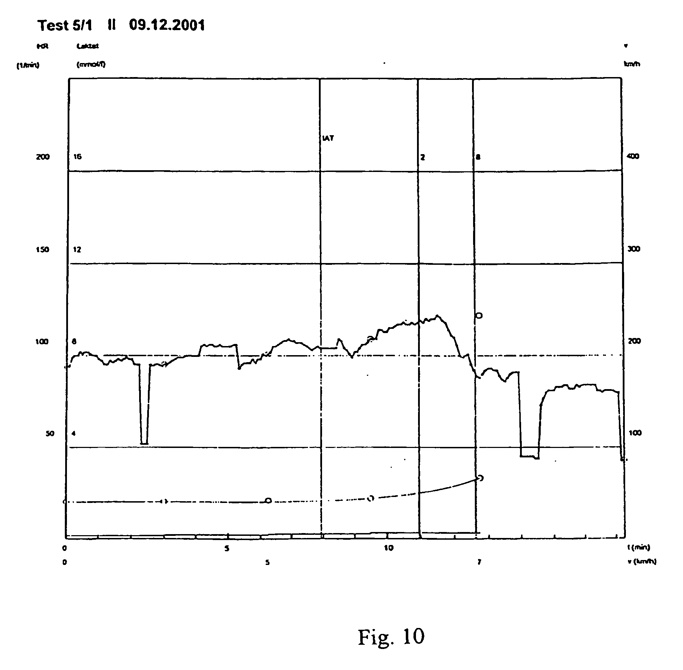

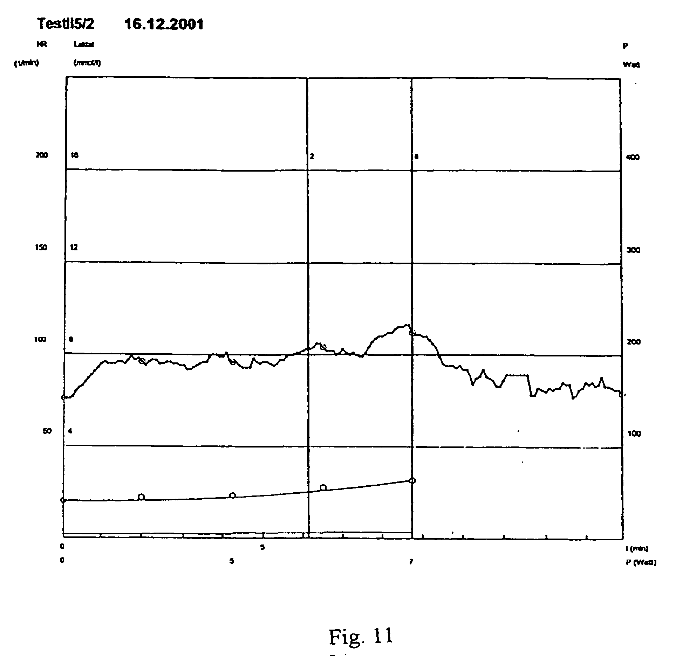

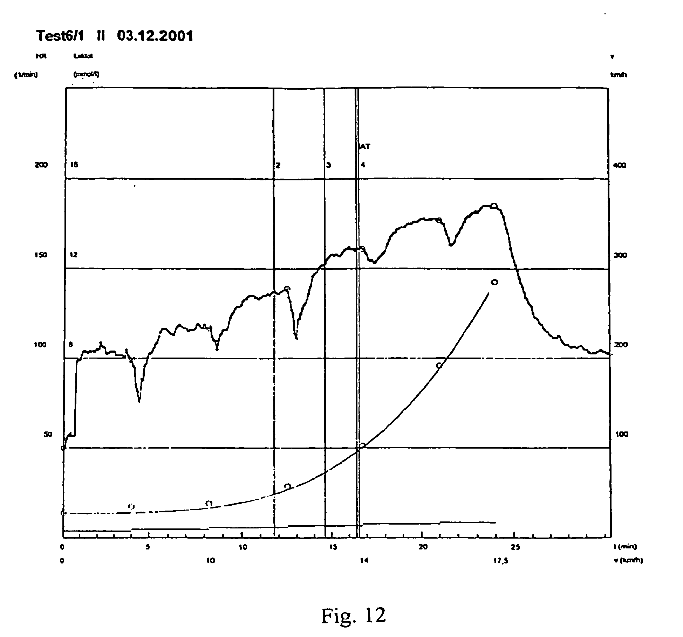

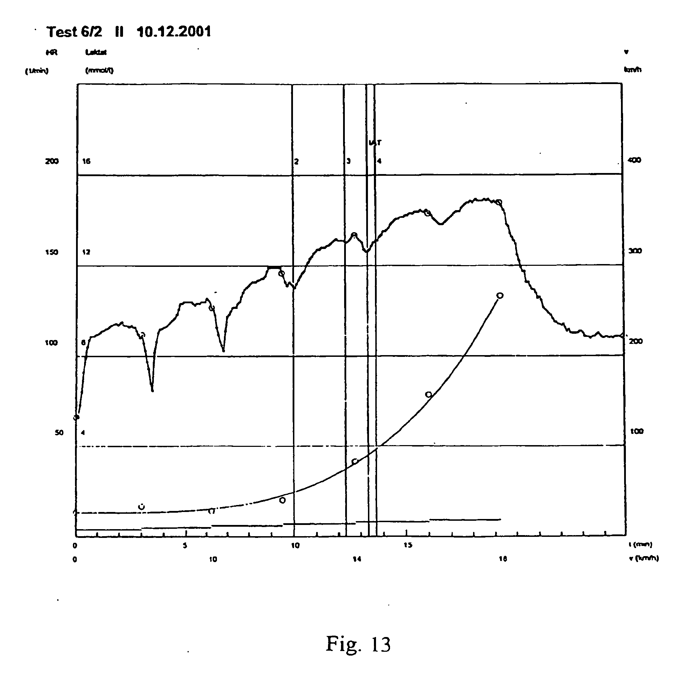

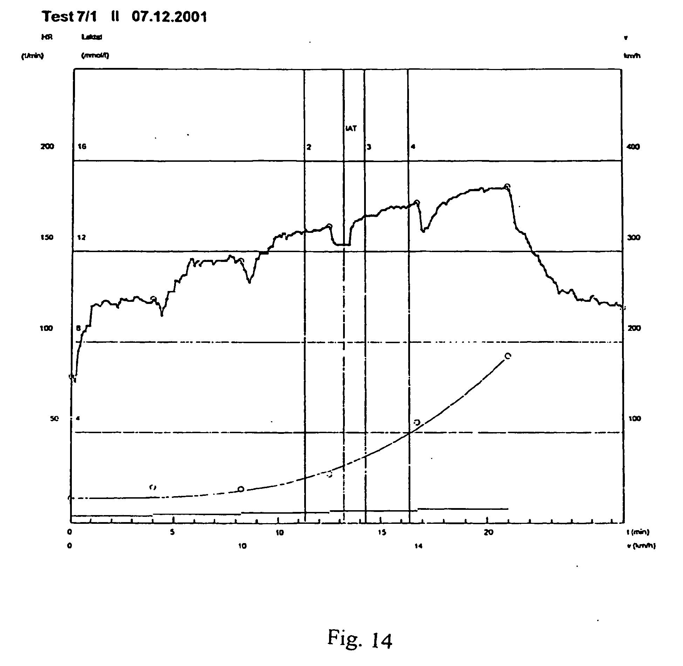

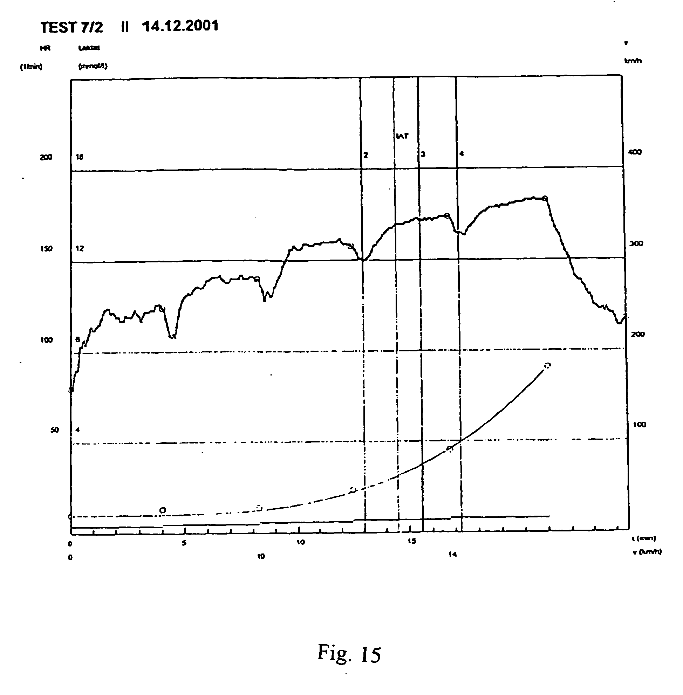

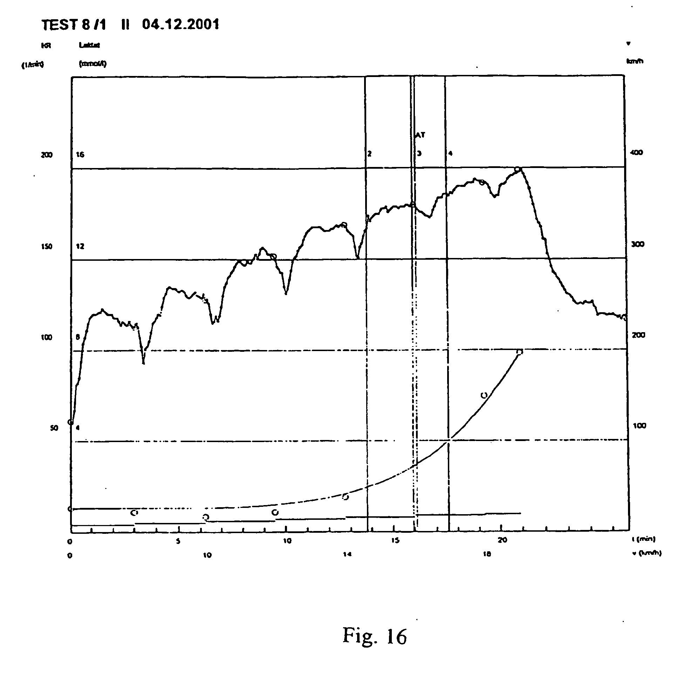

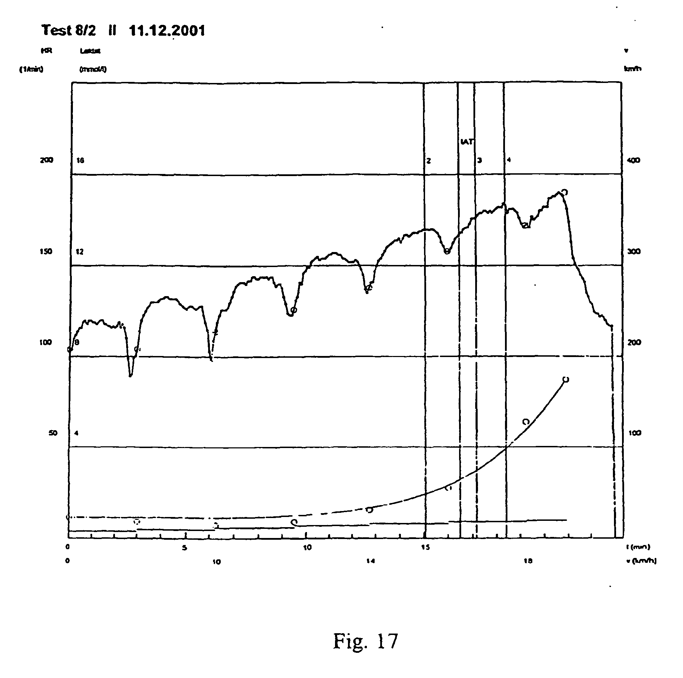

Bei einem ersten Versuch wurden 9 Personen unterschiedlichsten Alters und aus den verschiedensten Sportarten getestet. Davon konnten 8 Tests ausgewertet werden. Eine Testperson konnte den zweiten Test, der am Laufband ausgeführt wird, wegen einer Fußverletzung nicht mehr durchführen. Bei sieben von neun Testpersonen waren binnen einer Woche signifikante Senkungen des Lactatanteils im Blut zu erkennen. Das heißt, insbesondere für Sportler: Kürzere Regenerationszeit, längere Trainingseinheiten und somit einen schnellerer und besserer Trainingserfolg. Details zu den Testpersonen und die Testergebnisse sind in den nachfolgenden Tabellen 1 bis 23 angeführt. Aus den entsprechenden Figuren 2 bis 17 sind die Testverläufe ersichtlich, die die Wirkung der Verabreichung des Zeoliths belegen.

### Verabreichung - Dosierung:

Der Zeolith wird bevorzugt oral verabreicht, die Dosierung für Erwachsene liegt im Bereich zwischen 2 und 30 g, in extrem fein gemahlener Form auch darunter, bei gröberer Mahlung und extremer Anstrengung auch darüber. Die Verabreichung kann in Pulverform oder in Gelatinekapseln od.dergl. erfolgen, um einen geschützten Durchgang durch einen Teil des Verdauungssystems sicherzustellen. Die Auswirkungen dieser unterschiedlichen Verabreichungsform auf die Wirksamkeit sind noch nicht erforscht. Für Kinder sollten entsprechend dem Körpergewicht herabgesetzte Dosierungen angewandt werden. Nebenwirkungen oder unerwünschte Effekte konnten nicht festgestellt werden.

### Messergebnisse:

Die Probanden erhielten nach dem ersten der beiden Tests 3 mal täglich 2 Teelöffel, insgesamt somit etwa 12 g des oben beschriebenen Zeolithen, nämlich gemahlenen Klinoptilolith, oral verabreicht. Um eventuelle Beeinträchtigungen der Wirksamkeit beim Durchgang durch den Verdauungskanal zu kompensieren, wurde den Probanden auch 3 mal täglich 3 Gelatinekapseln mit jeweils 400 mg gemahlenen Klinoptilolith (genau das gleiche Material wie in Pulverform verabreicht), somit weitere 3,6 g, verabreicht.

Die Messergebnisse sind im folgenden für jeden der Probanden für zwei Tests angegeben. Dabei ist auch das wöchentliche Training des Probanden, versehen mit dem Vermerk "GA1" für "Grundlagenausdauer 1" und "GA2" für "Cardio- bzw. Herzkreislaufbelastung im Zuckerverbrennungsbereich", in der Sportmedizin übliche Bezeichnungen, angegeben. Die Diagramme zeigen die Herzfrequenz (oberste, stark schwankende Kurve), die Lactatbelastung (mittlere Kurve, gemittelt aus den einzelnen, durch kleine Kreise eingetragenen Messungen) und ganz unten die Angabe der Geschwindigkeitsstufen während des jeweiligen Tests. Aus dem Vergleich der jeweils beiden Tests geht die Wirkung des verabreichten Zeolithen deutlich hervor.

Ein weiterer Versuch wurde von Dr. med. Gerhard Stingl, einem Sportarzt in Klagenfurt, Österreich, an zehn Probanden durchgeführt. Dabei wurde jedem der Probanden während 3-er Monate regelmäßig Klinoptilolith wie oben erläutert, verabreicht und sichergestellt, dass die Trainings- und Ernährungsgewohnheiten in dieser Zeit nicht verändert wurden. Die Wirkung wurde am Laufbandergometer überprüft, die Laktatgewinnung erfolgte aus dem hyperämisierten rechten Ohrläppchen, die Messung wurde mit einem Biosen-Laktatmessgerät durchgeführt. Die Ergebnisse zeigten bei allen Probanden eine Absenkung des Laktatwertes bei jeder der Belastungsstufen und ein signifikantes Ansteigen der Leistungsfähigkeit.

### LACTAT-TEST BEZOGEN AUF DAS MEGAMIN

**TABELLE 1**

| Proband 1 | Test 1 | Test 2 | |
|---|---|---|---|
| Lactat 1 | 1,22 | 1,0 | |
| Lactat 2 | 1,38 | 0,9 | |
| Lactat 3 | 1,73 | 2,09 | |
| Lactat 4 | 2,19 | 2,74 | |
| Lactat 5 | 4,11 | 4,23 | |
| Lactat 6 | | | |
| Lactat 7 | | | |
| Lactat 8 | | | |
| Lactat 9 | | | |

| | | | |
|---|---|---|---|
| Training / Woche: 4 Stunden Eislaufen ( 2x2 ) = GA1 4 Stunden Hipp-Hopp ( 2x2 ) = GA1/2 5 Stunden Turnen | | | |

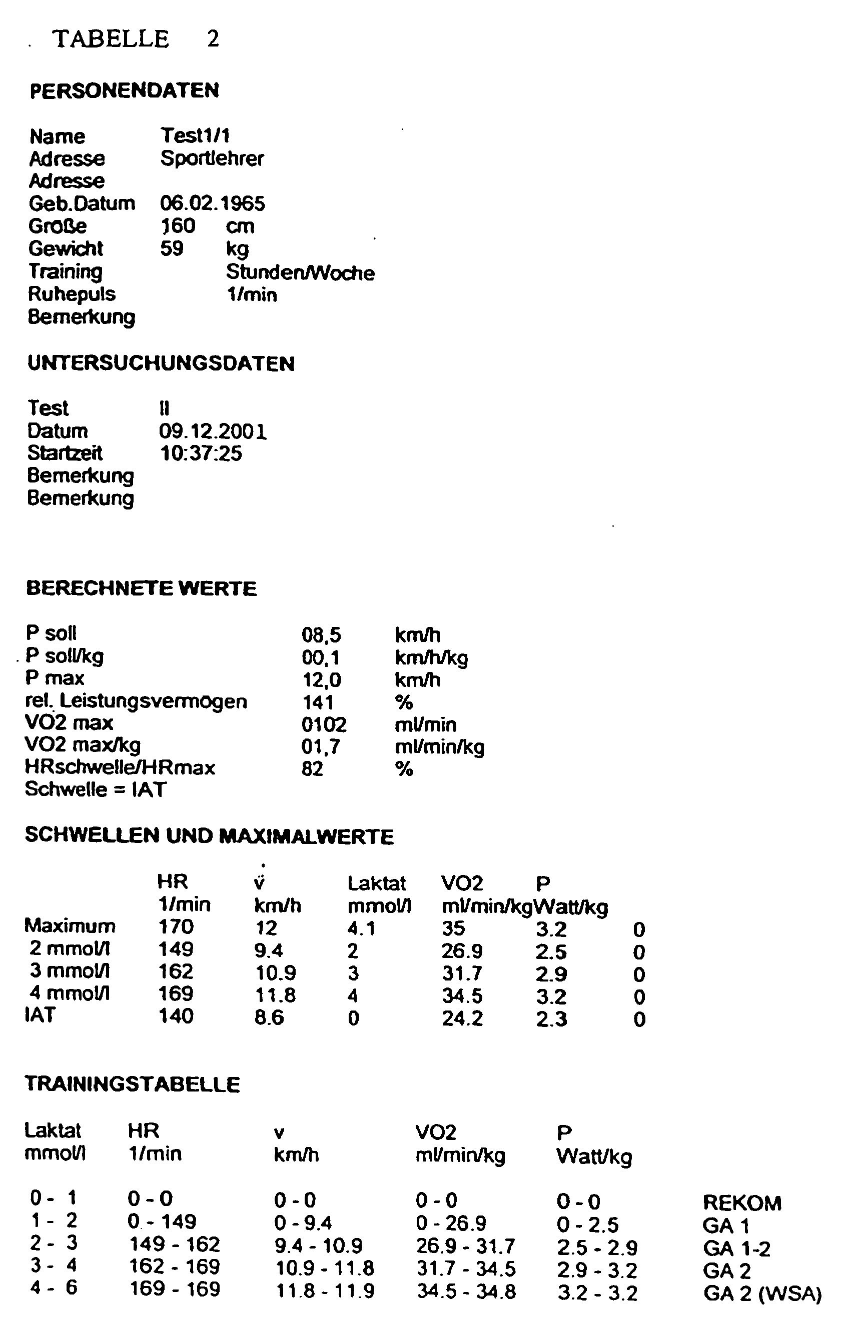

### LACTAT-TEST BEZOGEN AUF DAS MEGAMIN

**TABELLE 3**

| Proband 2 | Test 1 | Test 2 | |
|---|---|---|---|
| Lactat 1 | 1,14 | 0,99 | |
| Lactat 2 | 1,75 | 1,03 | |
| Lactat 3 | 2,45 | 2,29 | |
| Lactat 4 | 3,29 | 3,08 | |
| Lactat 5 | 4,19 | 4,15 | |
| Lactat 6 | 6,32 | 6,25 | |
| Lactat 7 | | | |
| Lactat 8 | | | |
| Lactat 9 | | | |

| | | | |
|---|---|---|---|
| Training / Woche: 6 Stunden GA1 12 Stunden GA1/2 | | | |

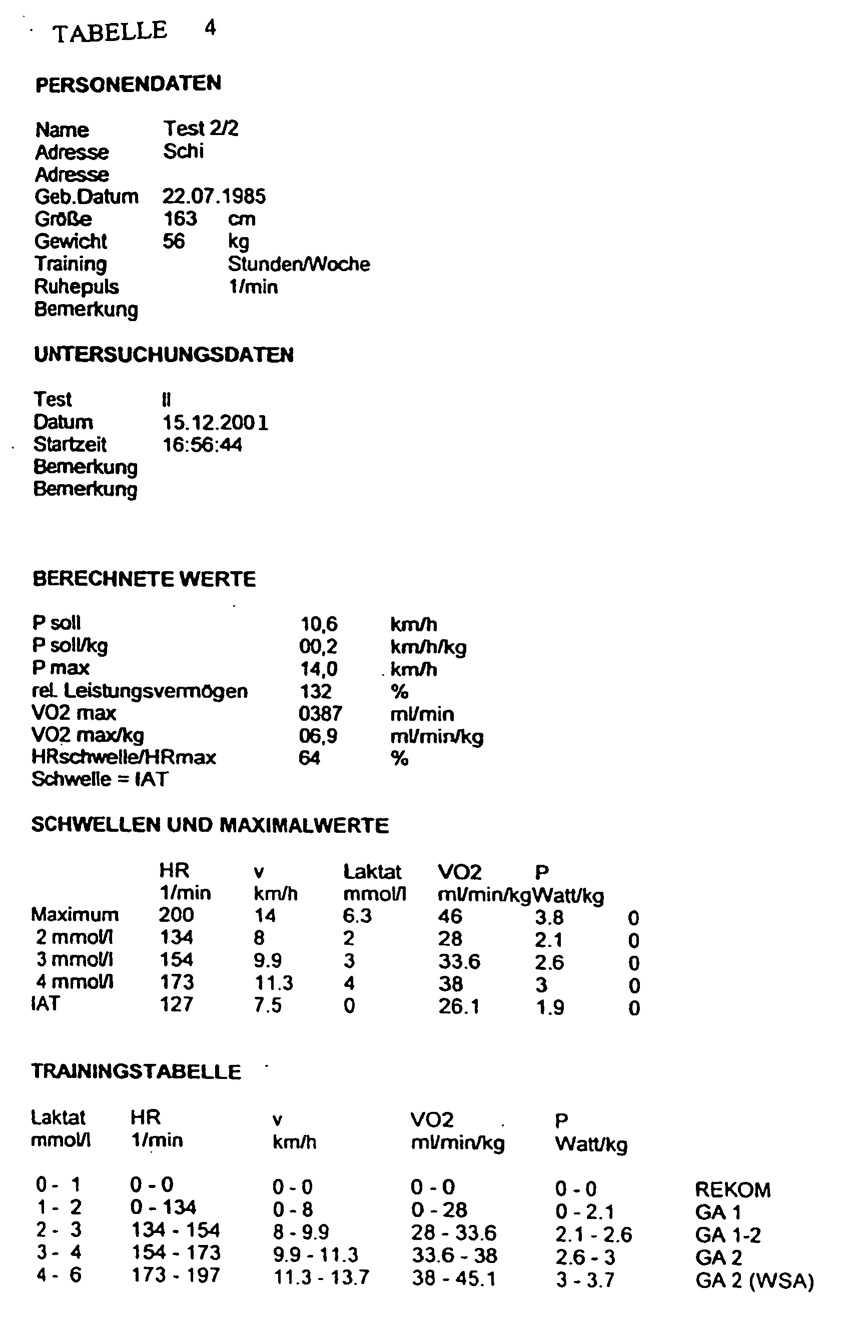

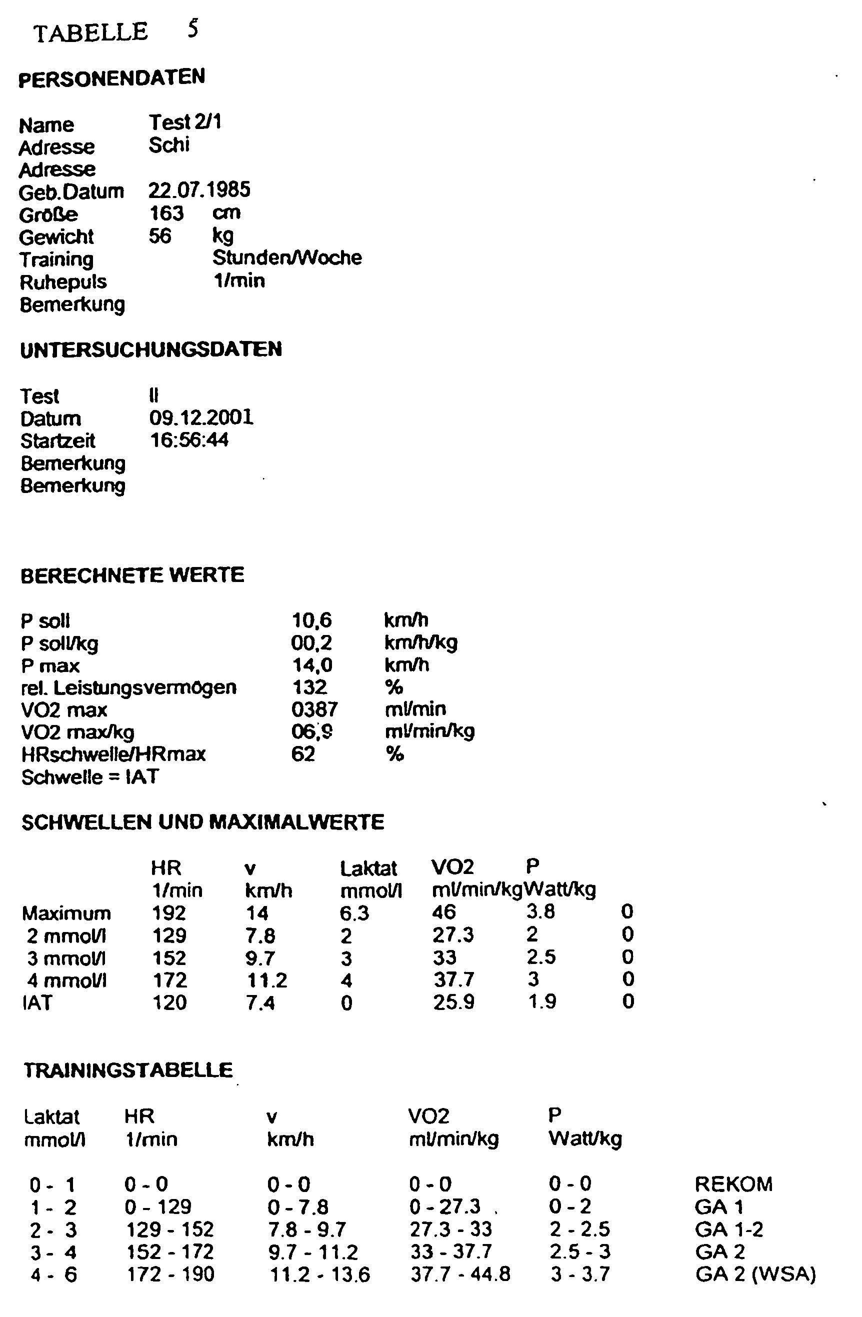

### LACTAT-TEST BEZOGEN AUF DAS MEGAMIN

**TABELLE 6**

| Proband 3 | Test 1 | Test 2 | |
|---|---|---|---|
| Lactat 1 | 0,8 | 0,76 | |
| Lactat 2 | 0,89 | 0,70 | |
| Lactat 3 | 1,92 | 1,24 | |
| Lactat 4 | 2,11 | 1,58 | |
| Lactat 5 | 2,31 | 1,73 | |
| Lactat 6 | 3,1 | 2,38 | |
| Lactat 7 | | | |
| Lactat 8 | | | |
| Lactat 9 | | | |

| | | | |
|---|---|---|---|
| Training/ Woche: 2 Stunden GA1 1 Stunde GA1/2 Schnee 4 Tage je 4 Stunden | | | |

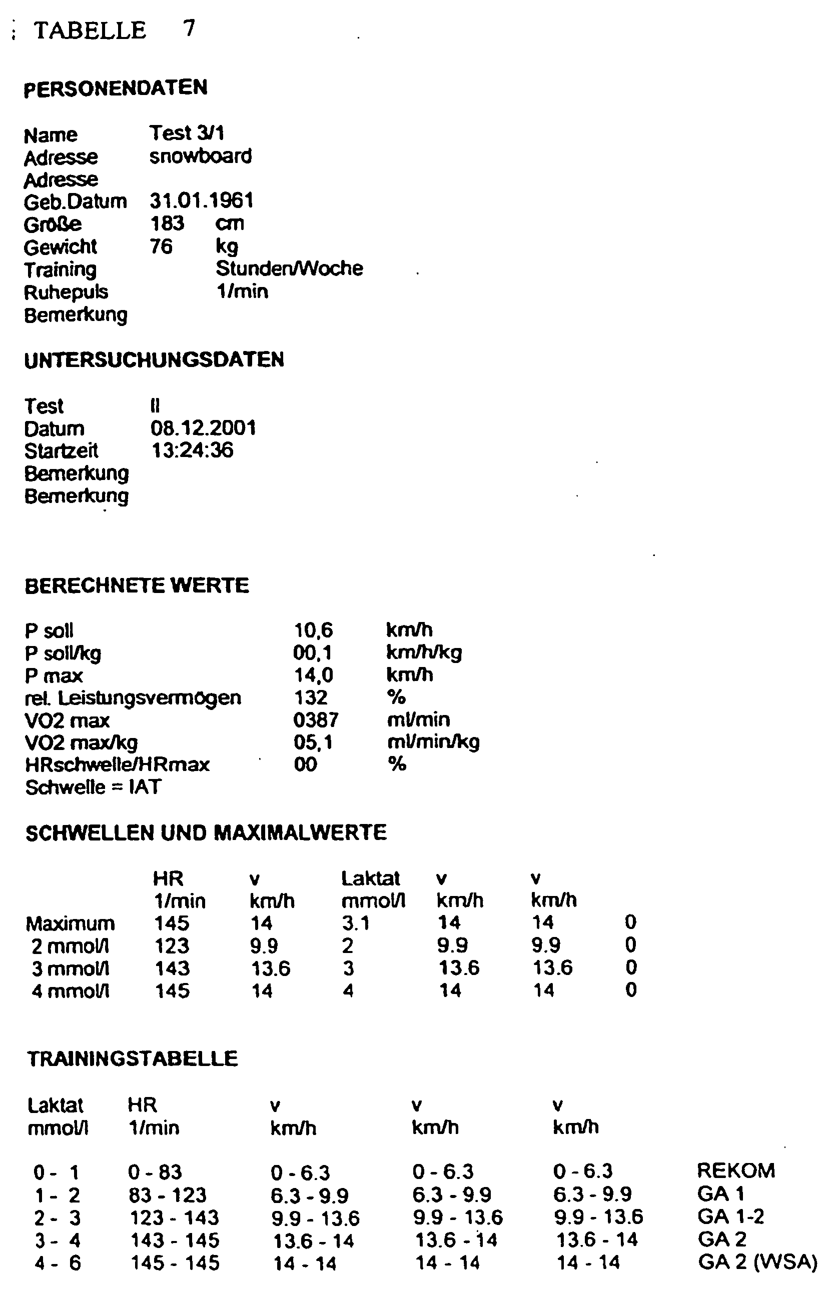

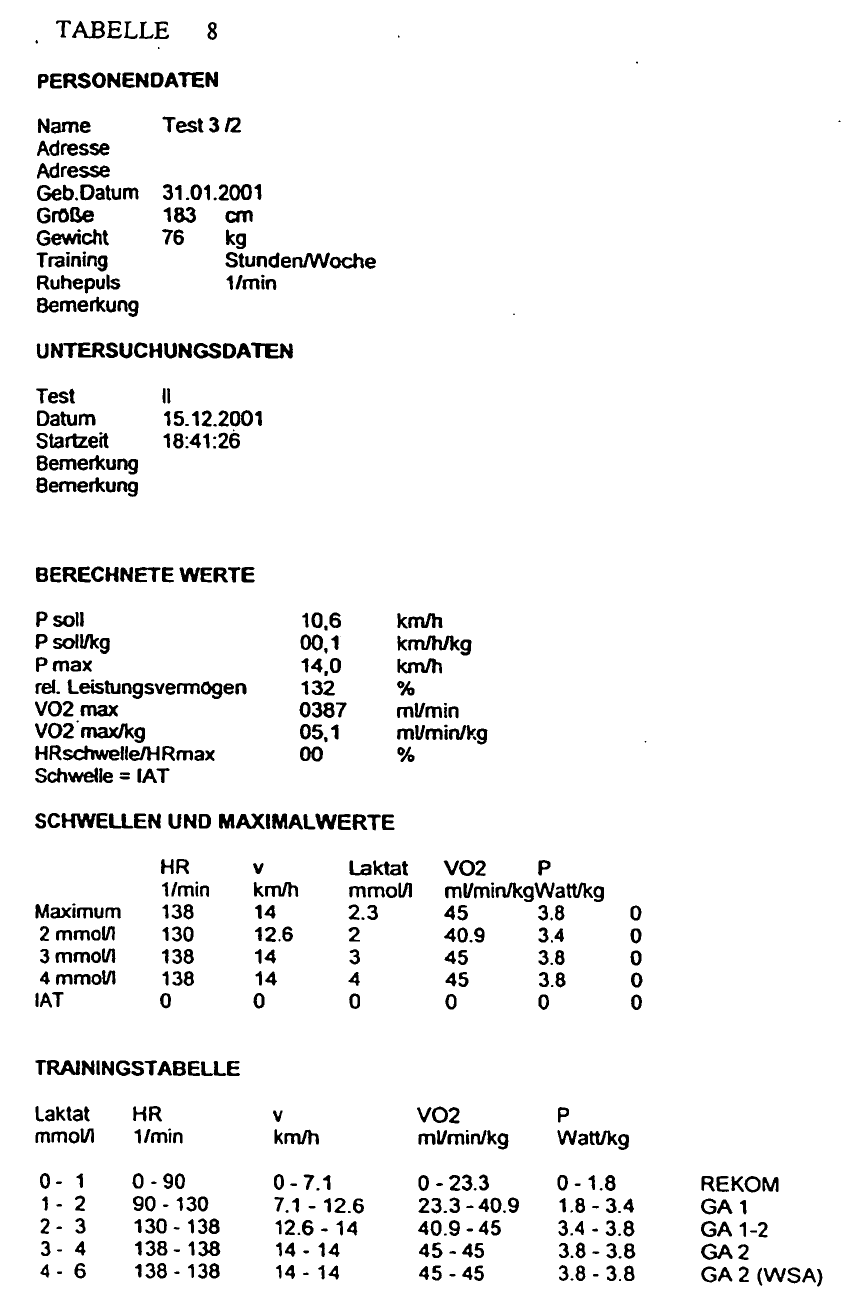

### LACTAT-TEST BEZOGEN AUF DAS MEGAMIN

**TABELLE 9**

| Proband 4 | Test 1 | Test 2 | |
|---|---|---|---|
| Lactat 1 | 0.88 | 0.79 | |
| Lactat 2 | 1,51 | 0,84 | |
| Lactat 3 | 1,61 | 0,94 | |
| Lactat 4 | 227 | 1,57 | |
| Lactat 5 | 3,27 | 2,62 | |
| Lactat 6 | | | |
| Lactat 7 | | | |
| Lactat 8 | | | |
| Lactat 9 | | | |

| | | | |
|---|---|---|---|
| Training / Woche: 2x2 Stunden GA1 2x45 Minuten GA1/2 1.Tag starker Durchfall Völlegefühl | | | |

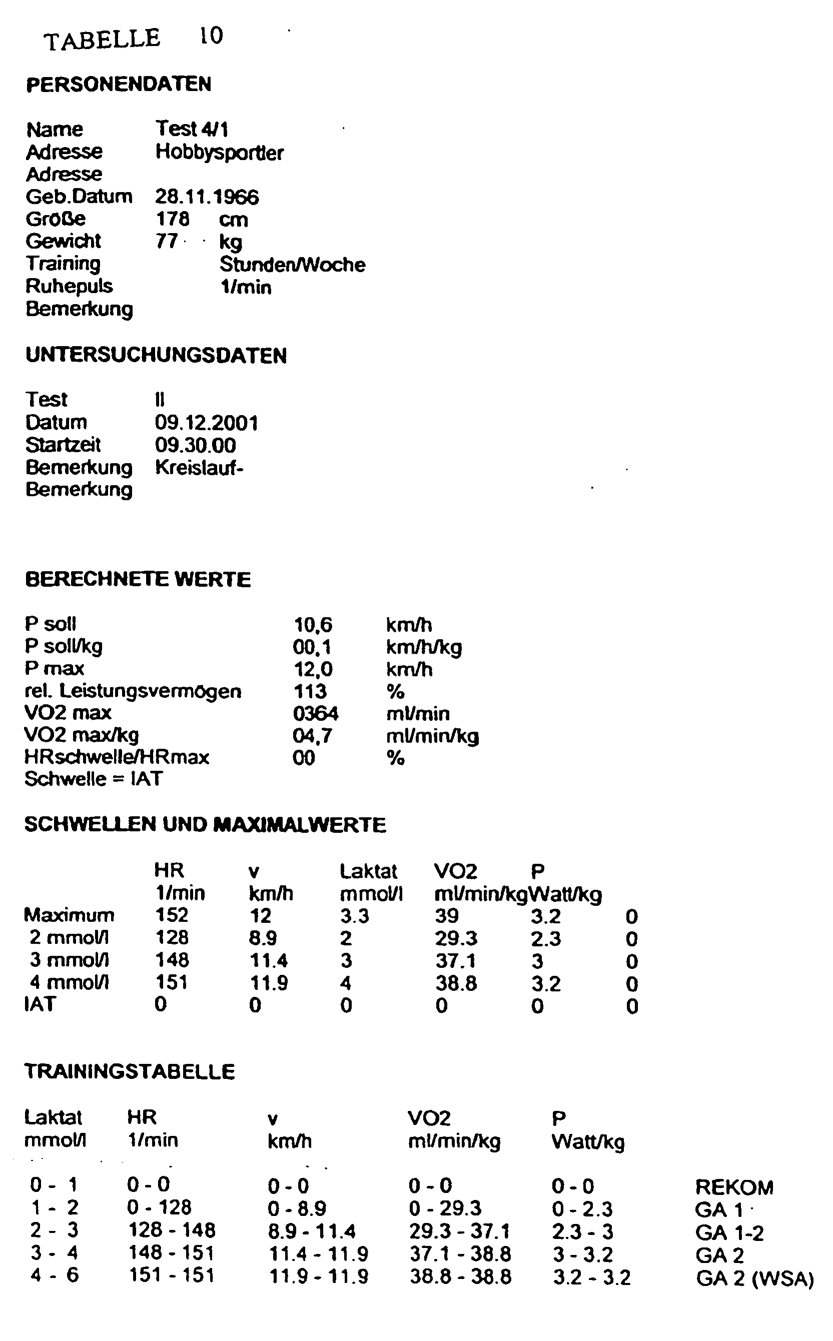

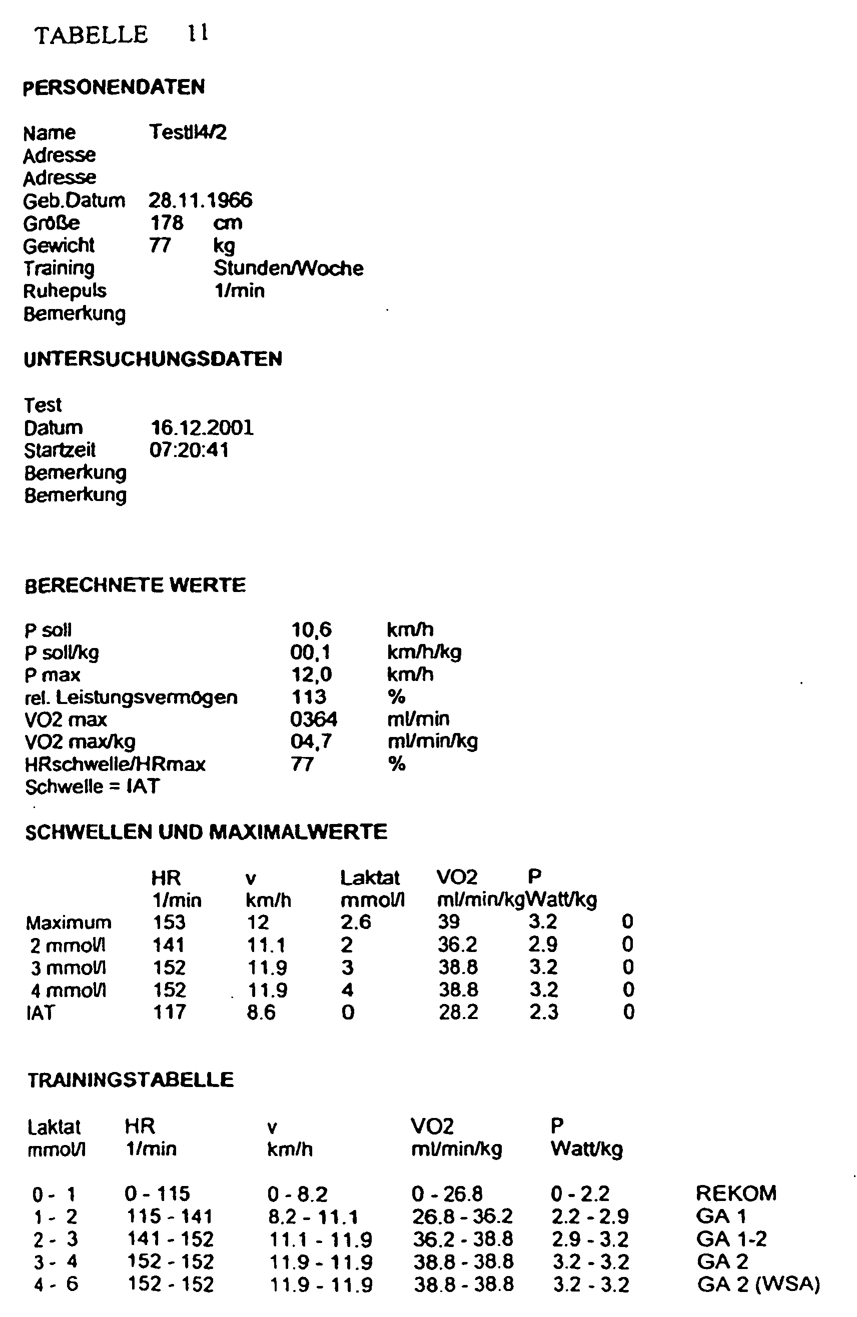

### LACTAT-TEST BEZOGEN AUF DAS MEGAMIN

**TABELLE 12**

| Proband 5 | Test 1 | Test 2 | |
|---|---|---|---|
| Lactat 1 | 1,62 | 1,60 | |
| Lactat 2 | 1,63 | 1,76 | |
| Lactat 3 | 1,69 | 1,85 | |
| Lactat 4 | 1,76 | 2,19 | |
| Lactat 5 | 2,67 | 2,52 | |
| Lactat 6 | | | |
| Lactat 7 | | | |
| Lactat 8 | | | |
| Lactat 9 | | | |

| | | | |
|---|---|---|---|
| Training / Woche : Keines kein Training, zu kalt Schlafstörung | | | |

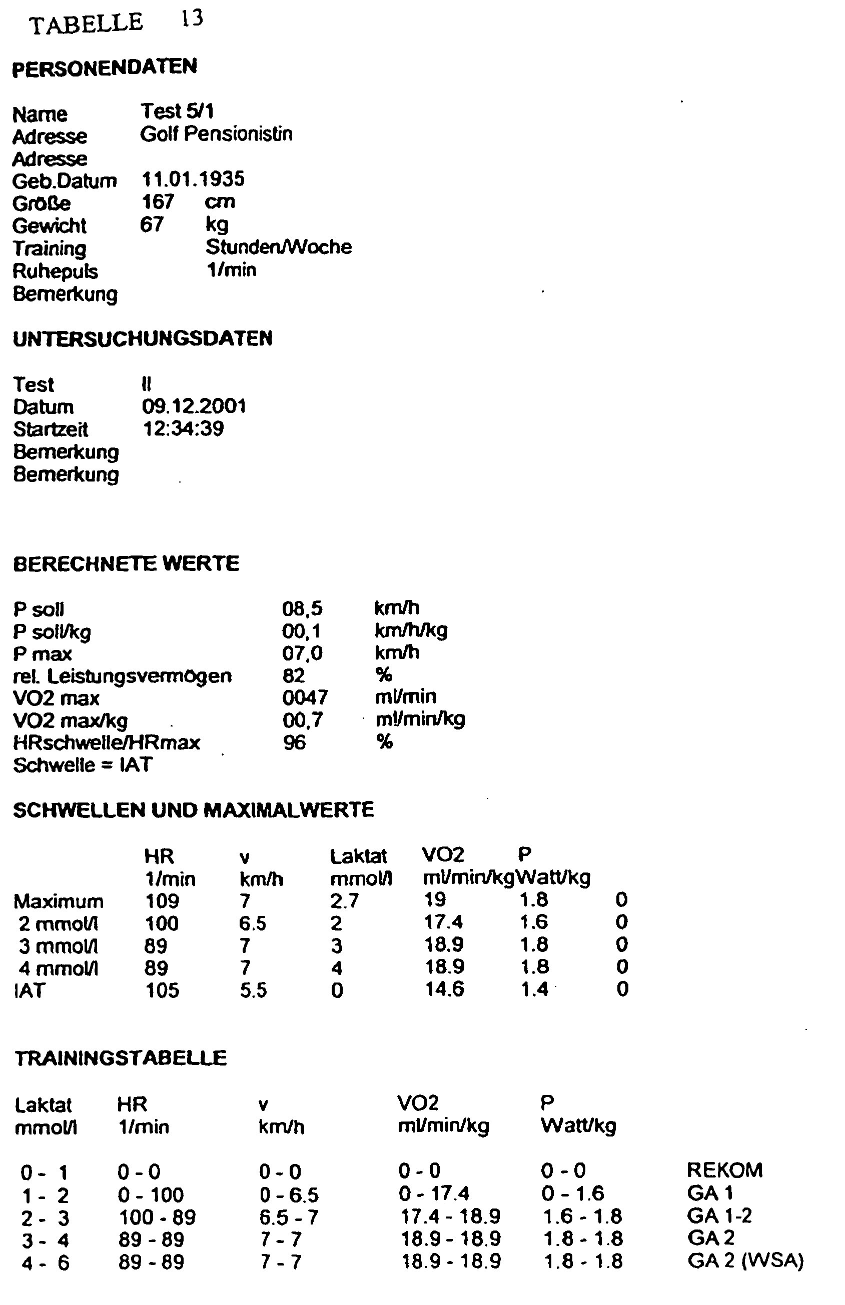

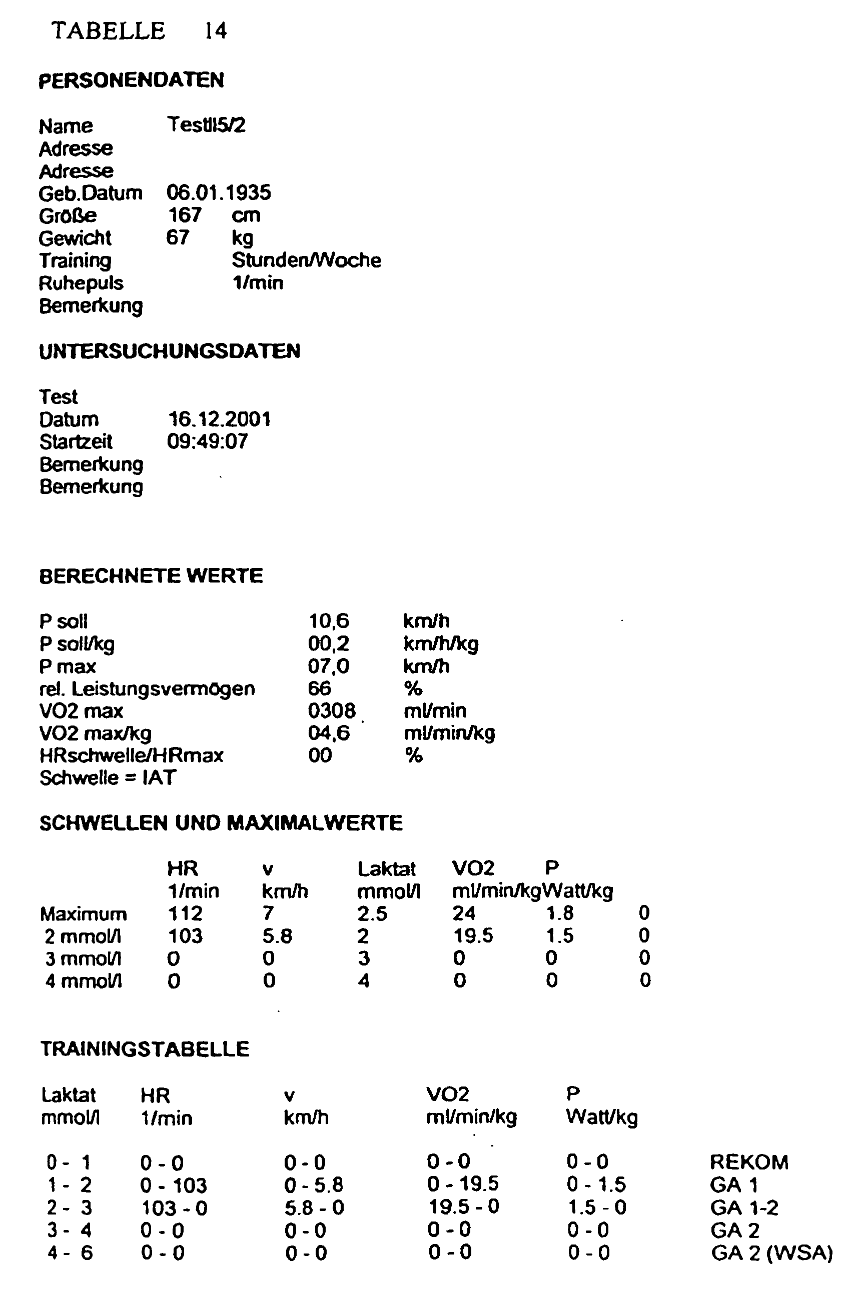

### LACTAT-TEST BEZOGEN AUF DAS MEGAMIN

**TABELLE 15**

| Proband 6 | Test 1 | Test 2 | |
|---|---|---|---|
| Lactat 1 | 1,11 | 1,07 | |
| Lactat 2 | 1,41 | 1,34 | |
| Lactat 3 | 1,55 | 1,44 | |
| Lactat 4 | 2,3 | 1,62 | |
| Lactat 5 | 4,11 | 3,32 | |
| Lactat 6 | 7,68 | 6,24 | |
| Lactat 7 | 11,41 | 10,67 | |
| Lactat 8 | | | |
| Lactat 9 | | | |

| | | | |
|---|---|---|---|
| Training / Woche: 3 x GA1 90 min 2x GA ½ 40 min | | | |

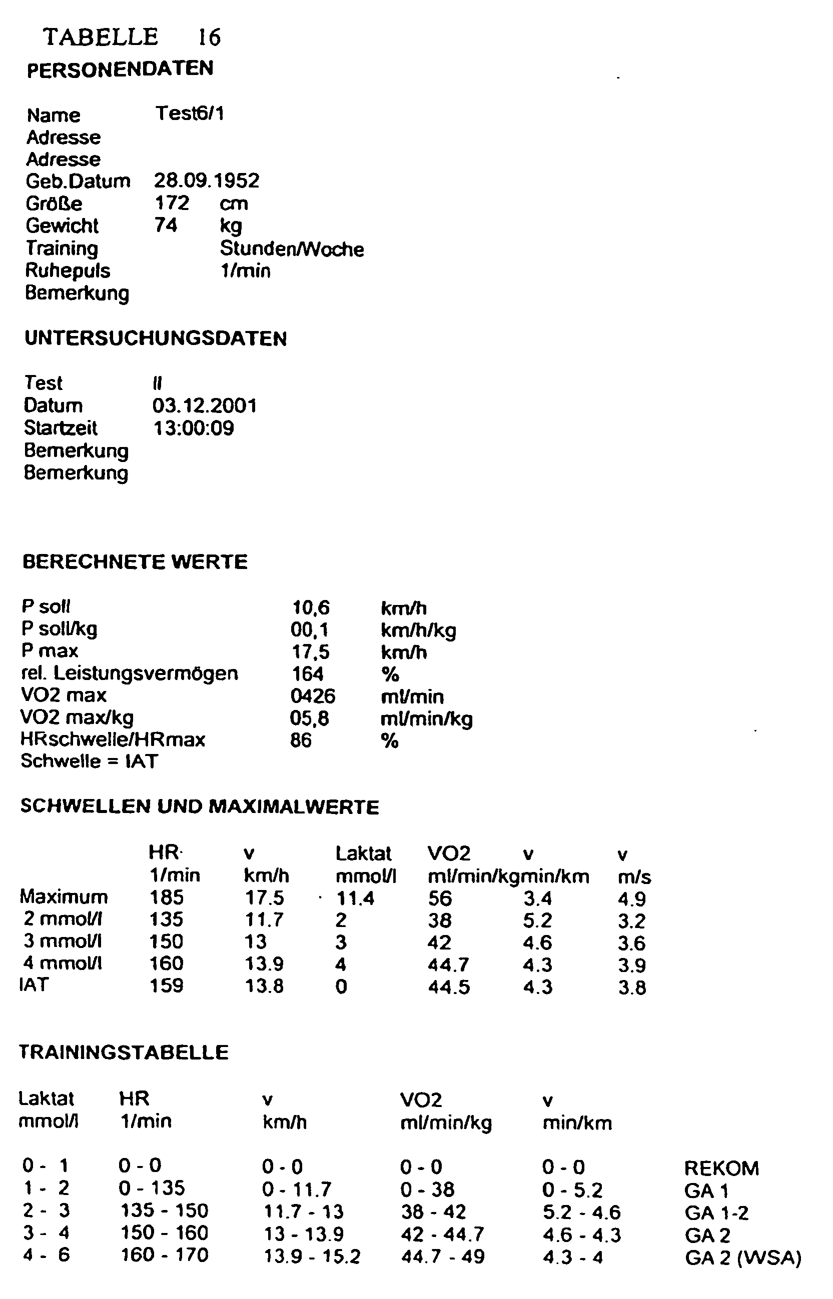

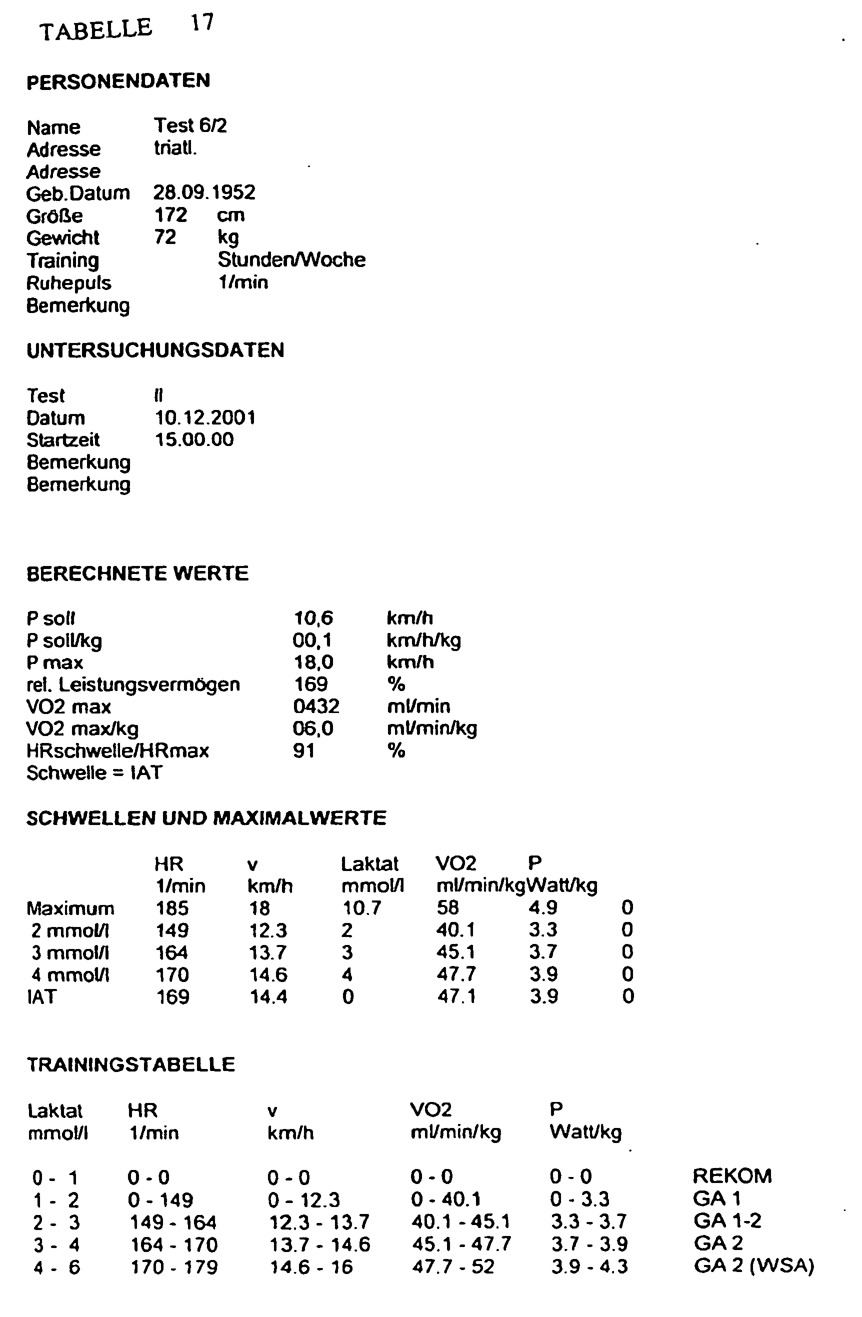

### LACTAT-TEST BEZOGEN AUF DAS MEGAMIN

**TABELLE 18**

| Proband 7 | Test 1 | Test 2 | |
|---|---|---|---|
| Lactat 1 | 1,11 | 0,80 | |
| Lactat 2 | 1,59 | 1,07 | |
| Lactat 3 | 1,51 | 1,14 | |
| Lactat 4 | 2,16 | 1,84 | |
| Lactat 5 | 4,64 | 3,64 | |
| Lactat 6 | 7,4 | 7,3 | |
| Lactat 7 | | | |
| Lactat 8 | | | |
| Lactat 9 | | | |

| | | | |
|---|---|---|---|
| Training / Woche: 3 x 90 min GA 1 1 x 60 min GA 1/2 | | | |

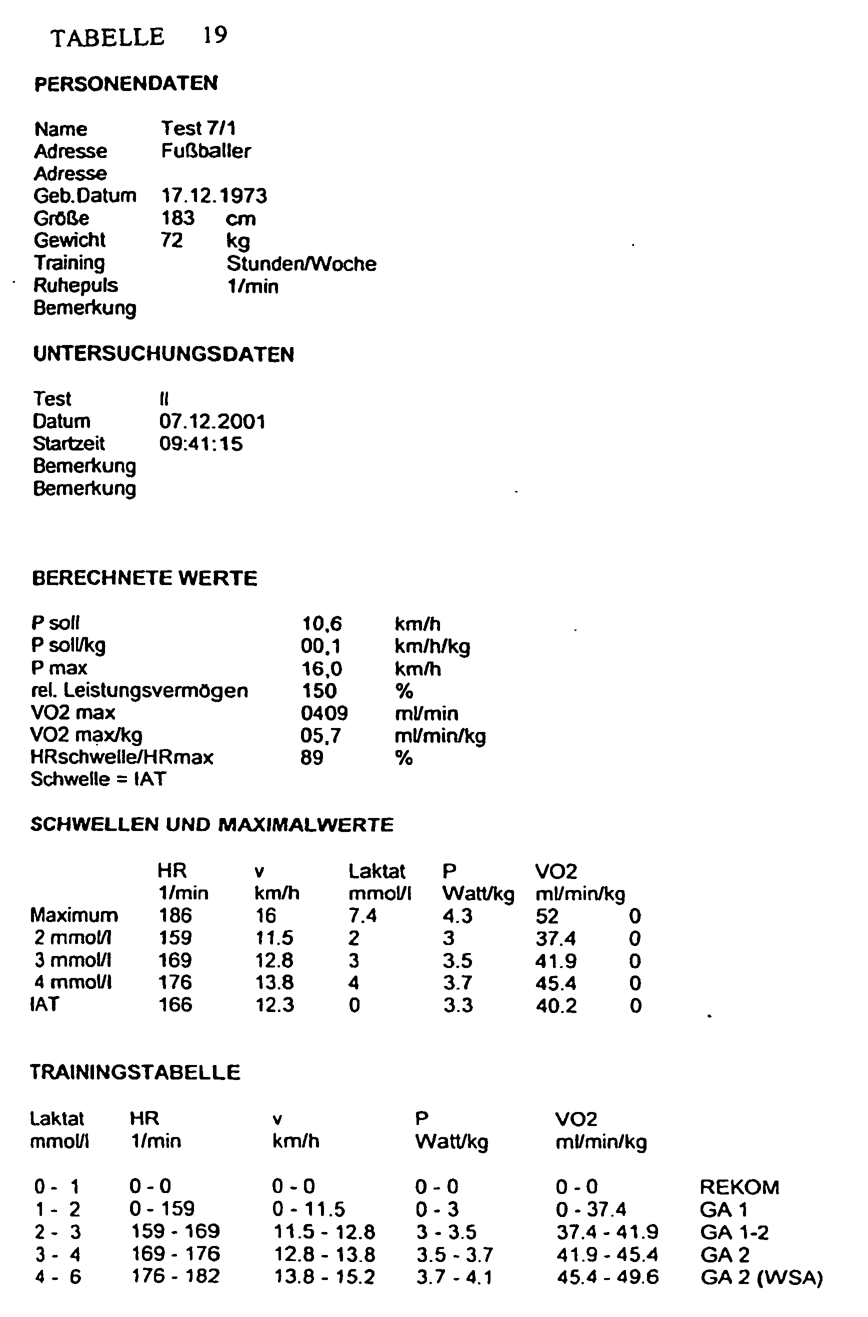

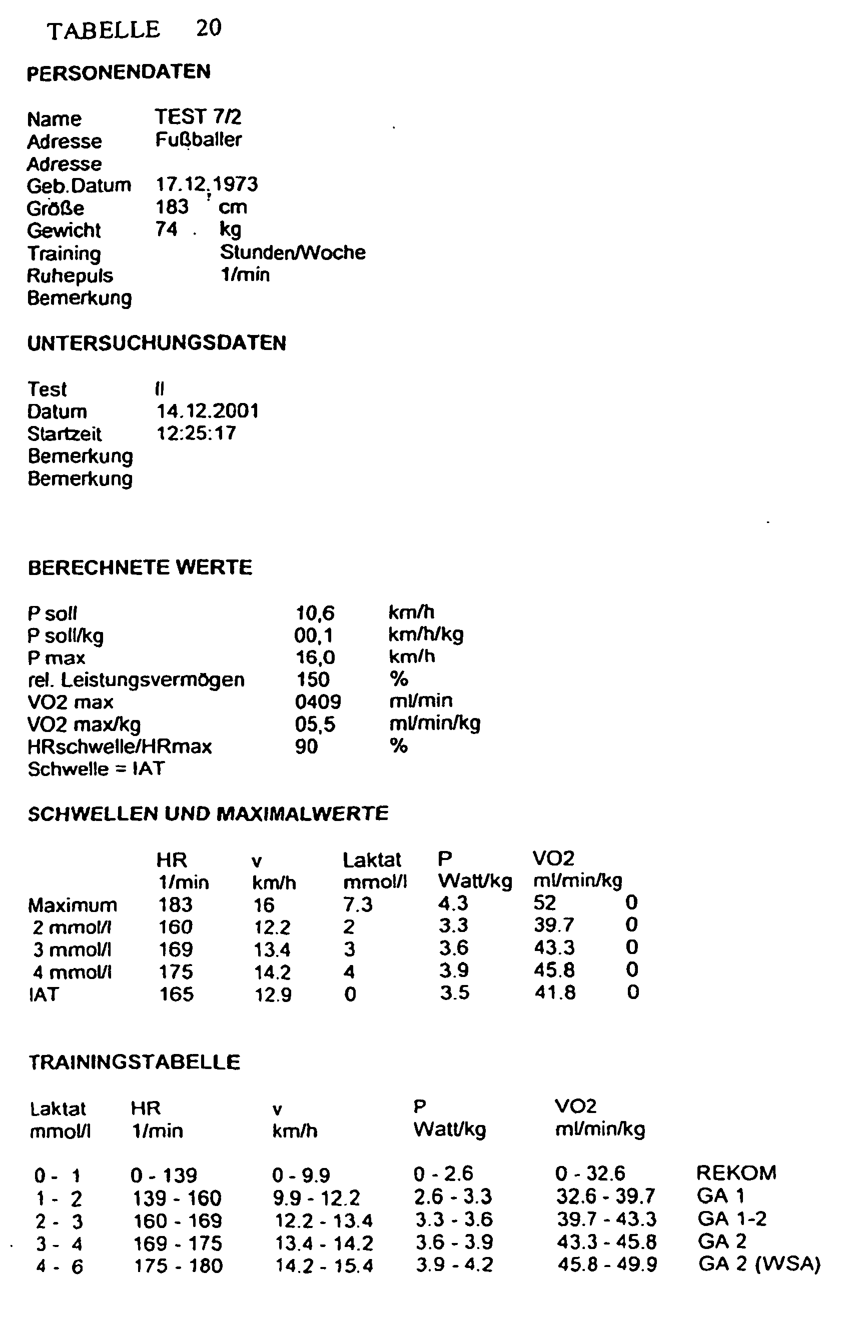

### LACTAT-TEST BEZOGEN AUF DAS MEGAMIN

**TABELLE 21**

| Proband 8 | Test 1 | Test 2 | |
|---|---|---|---|
| Lactat 1 | 1,06 | 0,93 | |
| Lactat 2 | 0,87 | 0,74 | |
| Lactat 3 | 0,68 | 0,6 | |
| Lactat 4 | 0,87 | 0,73 | |
| Lactat 5 | 1,52 | 1,24 | |
| Lactat 6 | 3,01 | 2,21 | |
| Lactat 7 | 5,99 | 5,12 | |
| Lactat 8 | 7,89 | 6,98 | |
| Lactat 9 | | | |

| | | | |
|---|---|---|---|
| Training / Woche : min 11 Stunden 1.Test leicht verkühlt | | | |

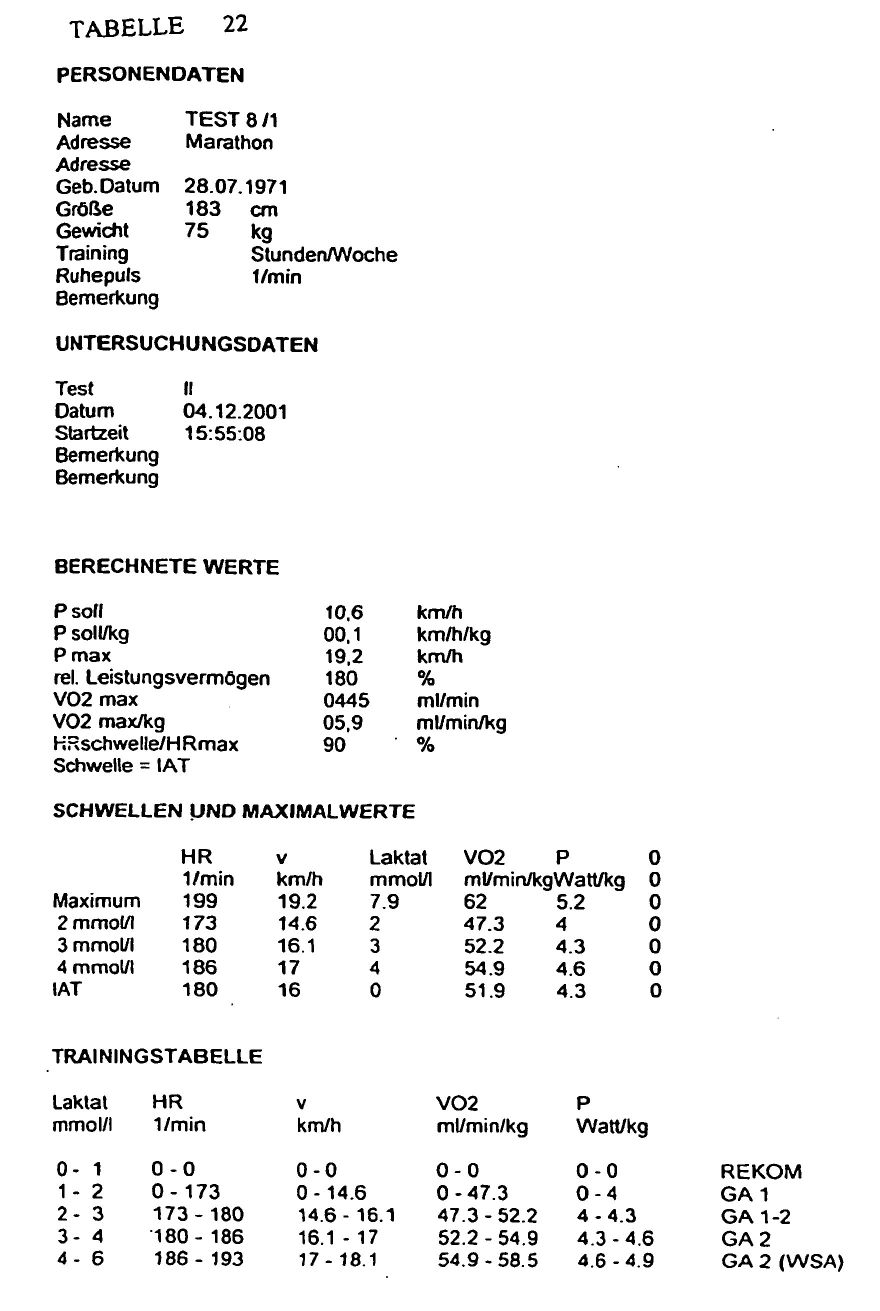

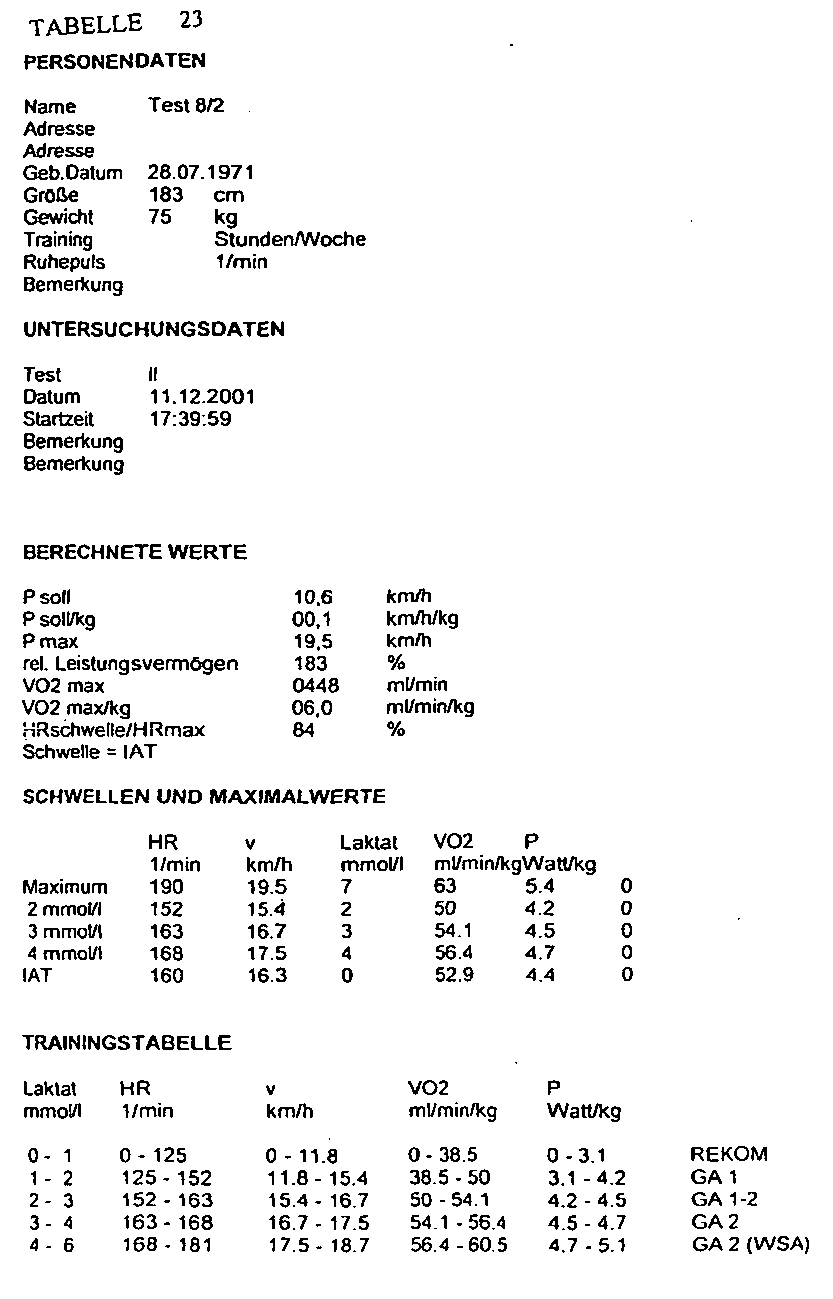

## Patentansprüche

1. Verwendung von Zeolithen zur Herstellung eines Verzehrproduktes bzw. eines oral zu verabreichenden Medikamentes zur Senkung der Lactatwerte im Blut von Säugetieren einschließlich Menschen.

2. Verwendung von Zeolithen nach Anspruch 1, **dadurch gekennzeichnet, dass** der Zeolith aus der Gruppe der natürlichen Zeolithe wie Klinoptilolith, Silberzeolith, Mordenit, Phillipsit, Analcim und/oder aus der Gruppe der künstlichen Zeolithe wie Zeolith A, Zeolith W, Zeolith X ausgewählt wird.

3. Verwendung von Zeolithen nach Anspruch 1 oder 2, **dadurch gekennzeichnet, dass** er Erwachsenen in einer täglichen Dosis von 2 bis 30 g, bevorzugt von etwa 12 g, verabreicht wird.

## Claims

1. Use of zeolites to manufacture a consumable product and/or a medicine to be administered orally for the reduction of lactate levels in the blood of mammals, including human beings,

2. Use of zeolites according to claim 1, **characterised in that** the zeolite is selected from the group of natural zeolites such as klinoptilolite, silver zeolite, mordenite, phillipsite and analcite, and/or from the group of artificial zeolites such as zeolite A, zeolite W and zeolite X.

3. Use of zeolites according to claim 1 or 2, **characterised in that** it is adminstered to adults in a daily dose of 2 to 30 g., preferably of about 12 g..

## Revendications

1. Utilisation de zéolithes pour la fabrication d'un produit alimentaire, respectivement d'un médicament à administrer par voie orale pour faire baisser le taux de lactate dans le sang de mammifères, y compris dans le sang de l'être humain.

2. Utilisation de zéolithes selon la revendication 1, **caractérisée en ce que** la zéolithe est choisie parmi le groupe constitué des zéolithes naturelles telles que la clinoptilolithe, la zéolithe argentée, la mordénite, la philipsite, l'analcime et/ou parmi le groupe constitué par les zéolithes artificielles telles que la zéolithe A, la zéolithe W et la zéolithe X.

3. Utilisation de zéolithes selon la revendication 1 ou 2, **caractérisée en ce qu'**elles sont administrées à des adultes selon une dose quotidienne allant de 2 à 30 g, de préférence de 12 g environ.
